# EUROPEAN PATENT APPLICATION

(11) **EP 4 524 655 A1**
(43) Date of publication of application: **19.03.2025**
(21) Application number: 24194132.7
(22) Date of filing: 12.08.2024
(51) Int. Cl.: G03F 7/09, G03F 7/11

(54) **COMPOUND FOR FORMING METAL-CONTAINING FILM, COMPOSITION FOR FORMING METAL-CONTAINING FILM, AND PATTERNING PROCESS**

(30) Priority: 29.08.2023 JP 2023139356
(71) Applicant: SHIN-ETSU CHEMICAL CO., LTD., Tokyo 1000005 (JP)
(72) Inventor: KOBAYASHI, Naoki, Niigata (JP); NAGAMACHI, Nobuhiro, Niigata (JP); KORI, Daisuke, Niigata (JP); ISHIWATA, Kenta, Niigata (JP)
(74) Representative: Sonnenhauser, Thomas Martin

(57) **Abstract**

The present invention aims to provide: a compound for forming a metal-containing film that yields a resist middle layer film enabling to obtain a favorable pattern shape and having high adhesiveness to a resist upper layer film to prevent collapse of a fine pattern in a fine patterning process in a semiconductor device manufacturing process; a composition for forming a metal-containing film using the compound; and a patterning process using the composition. A compound for forming a metal-containing film contains: at least one metal atom selected from a group consisting of Ti, Zr, and Hf; and a multidentate ligand coordinated to the metal atom and containing a cyclic ether structure having 2 to 13 carbon atoms

## Description

### TECHNICAL FIELD

The present invention relates to a compound for forming a metal-containing film, a composition for forming a metal-containing film using the compound, and a patterning process using the composition, that are usable for fine patterning by a multilayer resist method in a semiconductor device manufacturing process.

### BACKGROUND ART

As higher integration and speed are achieved in LSI, miniaturization of pattern size progresses rapidly. Along with this miniaturization, lithography techniques have achieved formation of a fine pattern by shortening a wavelength of a light source and appropriately selecting a resist composition corresponding thereto. A positive photoresist composition used in a single layer has been the key to this achievement. This single-layer positive photoresist composition contains a resist resin, which has a skeleton having etching resistance against dry etching with chlorine-based or fluorine-based gas plasma and a switching mechanism to cause dissolution of an exposed portion such that the exposed portion is dissolved to form a pattern, and a substrate to be processed is processed by dry etching while the remained resist pattern is used as an etching mask.

However, miniaturing, that is, reducing pattern width while maintaining film thickness of a photoresist film for use has caused a problem of degraded resolution performance of the photoresist film, and pattern development of the photoresist film with a developer has caused a problem of a so-called high aspect ratio, resulting in pattern collapse. Therefore, thickness of the photoresist film has been decreased as the pattern has been miniaturized.

Meanwhile, commonly used for processing a substrate to be processed is a method of processing a substrate by dry etching while using a photoresist film having a formed pattern as an etching mask. However, in reality, there is no dry etching method capable of securing complete etch selectivity between the photoresist film and the substrate to be processed. Hence, there has been a problem that the photoresist film is also damaged and collapses during processing of the substrate, making it impossible to accurately transfer a resist pattern to the substrate to be processed. Accordingly, as miniaturization of a pattern has progressed, higher dry etching resistance has been required for a photoresist composition. On the other hand, a resin used in the photoresist composition has been required to absorb less light at an exposure wavelength to enhance resolution. Therefore, as exposure light has been changed to the one having a shorter wavelength, such as i-line, KrF, and ArF, the resin also has been changed to a novolac resin, polyhydroxystyrene, and a resin having an aliphatic polycyclic skeleton. However, in reality, an etch rate has been increased under dry etching conditions during processing of a substrate, and recent photoresist compositions having high resolution tend to have rather weak etching resistance.

Accordingly, a substrate to be processed should be processed by dry etching with a thinner photoresist film having weaker etching resistance, and it has become an urgent matter to secure a material and a process for this processing step.

One of methods to solve such problems is a multilayer resist method. This method includes: interposing a resist middle layer film having different etch selectivity from that of a photoresist film (i.e. a resist upper layer film) between the resist upper layer film and a substrate to be processed; giving a pattern in the resist upper layer film, followed by transferring the pattern to the resist middle layer film by dry etching while using the resist upper layer film pattern as a dry etching mask; and further transferring the pattern to the substrate to be processed by dry etching while using the resist middle layer film as a dry etching mask.

One of the multilayer resist methods is a three-layer resist method that can be performed by using a common resist composition used in a single-layer resist method. In this three-layer resist method, for example, an organic film of a novolac resin or the like is formed on a substrate to be processed as a resist underlayer film; a silicon-containing resist middle layer film is formed thereon as a resist middle layer film; and a common organic photoresist film is formed thereon as a resist upper layer film. When dry etching is performed with fluorine-based gas plasma, the organic resist upper layer film can have good etch selectivity to the silicon-containing resist middle layer film and thus, the resist upper layer film pattern can be transferred to the silicon-containing resist middle layer film by dry etching with the fluorine-based gas plasma. According to this method, the pattern can be transferred to the silicon-containing resist middle layer film (resist middle layer film) even by using a resist composition which makes it difficult to form a pattern having film thickness sufficient for directly processing the substrate to be processed or a resist composition having dry etching resistance insufficient for processing the substrate. Subsequently transferring the pattern by dry etching with oxygen-based or hydrogen-based gas plasma can yield an organic film (resist underlayer film) pattern of the novolac resin or the like having dry etching resistance sufficient for processing the substrate. As the aforementioned resist underlayer film, many materials such as those described in Patent Document 1, for example, have already been known.

Used as the silicon-containing resist middle layer film used in the three-layer resist method as described above is: a silicon-containing inorganic film by CVD, for example, SiO₂ film (for example, Patent Document 2) and SiON film (for example, Patent Document 3); a film obtained by spin coating such as SOG (spin-on glass) film (for example, Patent Document 4, Non Patent Document 1) and a crosslinkable silsesquioxane film (for example, Patent Document 5); and the like, and probably a polysilane film (for example, Patent Document 6) is also usable. Among them, the SiO₂ film and SiON film have high performance as a dry etching mask upon dry etching of an underlying organic film, but a special apparatus is required for deposition thereof. In contrast, the SOG film, crosslinkable silsesquioxane film, and polysilane film can be formed only by spin coating and heating so as to be considered to have high process efficiency.

The silicon-containing film conventionally used for such multilayer resist method has some problems. For example, it is well known that when a resist pattern is intended to be formed by photolithography, exposure light is reflected from a substrate and interferes with incident light, causing a problem of so-called standing waves. To obtain a fine pattern with no edge roughness on a resist film by state-of-the-art ArF immersion/High-NA exposure conditions, an antireflection function is essential for a middle layer film. Furthermore, in the state-of-the-art semiconductor process as described above, a photoresist becomes thinner and thinner so that the middle layer film is also required to be thinner, and in the next generation exposure process, it is required to impart an antireflective effect with film thickness of 30 nm or less. Furthermore, a lower dry etch rate in oxygen gas plasma generally used for processing a resist underlayer film is preferable to enhance etching selectivity of the middle layer film and the underlayer film, and improvement of dry etching resistance has been required for the middle layer film due to the trend toward thinner films.

As the resist middle layer film satisfying the requirements for such antireflective effect and dry etching characteristics, a metal hard mask film containing Ti or Zr, instead of the conventional silicon-containing film, attracts attention. TiO₂ and ZrO₂ are known as a material having a high refractive index, and it is possible to enhance the antireflective effect under the High-NA exposure conditions by containing them in a film. Additionally, due to a contained metal-oxygen bond, excellent dry etching resistance to an oxygen gas can be expected.

Furthermore, the metal hard mask film has excellent dry etching resistance not only to an oxygen gas but also to a fluorine gas, making it promising also in a two-layer resist method including depositing the metal hard mask film on a substrate to be processed as a resist underlayer film and forming a resist upper layer film thereon.

Meanwhile, when such metal hard mask film is used directly under a resist upper layer film, there is a problem of adhesiveness to a resist pattern. The cured metal hard mask film has much higher surface energy (or a smaller water contact angle) than that of a subsequently applied photoresist. Such mismatch of the surface energy causes poor adhesion between the metal hard mask film and the subsequently applied photoresist, resulting in pattern collapse.

To prevent the photoresist pattern collapse on the metal hard mask film, surface modification of the metal hard mask film is required. For example, Patent Document 7 reports the metal hard mask containing a surface modified organic polymer. It is reported that adhesiveness to the resist pattern is improved by utilizing difference of free energy between an organic polymer and a metal compound to localize the organic polymer in a surface layer. To prevent the pattern collapse, an organic polymer containing a surface process portion selected from hydroxyl, protected hydroxyl, protected carboxyl, and a mixture thereof is used. However, in the current situation requiring finer pattern formation, these materials are not considered to have enough performance to prevent the pattern collapse. Furthermore, there is a risk of deterioration in dry etching resistance to an oxygen gas because of the contained organic polymer, and thus it has been demanded to develop a compound for forming a metal-containing film having excellent adhesiveness to a resist upper layer film.

Recently, it is considered that interaction at an interface between a resist upper layer film and an underlayer film directly below the resist upper layer film in a fine pattern also affects sensitivity of the resist, pattern shape (rectangularity and residues in space portions), etc. In view of these as well, improvement to the performance of the underlayer film directly below the resist upper layer film is required (Non Patent Document 2).

### CITATION LIST

### PATENT LITERATURE

Patent Document 1: JP 2004-205685 A
Patent Document 2: JP H07-183194 A
Patent Document 3: JP H07-181688 A
Patent Document 4: JP H05-291208 A
Patent Document 5: JP 2005-520354 A
Patent Document 6: JP H11-60735 A
Patent Document 7: JP 6463600 B

### NON PATENT LITERATURE

Non Patent Document 1: J. Appl. Polym. Sci., Vol. 88, 636-640 (2003)
Non Patent Document 2: Proc. SPIE Vol. 7273, 72731J (2009)

### SUMMARY OF INVENTION

### TECHNICAL PROBLEM

The present invention has been made in view of the above circumstances and aims to provide: a compound for forming a metal-containing film that yields a metal-containing film enabling to obtain a favorable pattern shape and having high adhesiveness to a resist upper layer film to prevent collapse of a fine pattern in a fine patterning process in a semiconductor device manufacturing process; a composition for forming a metal-containing film using the compound; and a patterning process using the composition.

### SOLUTION TO PROBLEM

To solve the above problems, the present invention provides a compound for forming a metal-containing film, containing: at least one metal atom selected from a group consisting of Ti, Zr, and Hf; and a multidentate ligand coordinated to the metal atom and containing a cyclic ether structure having 2 to 13 carbon atoms.

By using such compound for forming a metal-containing film in a composition for forming a resist underlayer film, it is possible to form a metal-containing film having high adhesiveness to a resist upper layer film and an effect of preventing collapse of a fine pattern, as well as enabling to obtain a favorable pattern shape. Furthermore, the metal-containing film thus obtained shows a high refractive index such that it can achieve a high antireflective effect in ArF immersion/under High-NA exposure conditions.

Furthermore, the multidentate ligand preferably has a structure represented by the following general formulae (1-A) to (1-D), 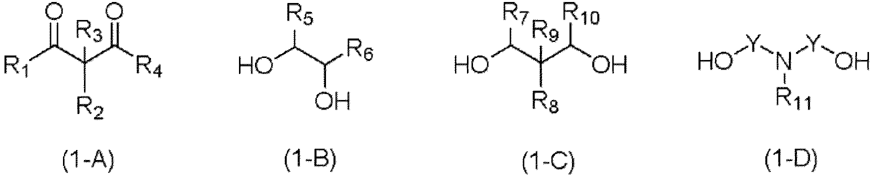 in the above general formulae (1-A) to (1-D), R₁ to R₄ represent a hydrogen atom or a monovalent organic group having 1 to 20 carbon atoms, which optionally contains a cyclic ether structure having 2 to 13 carbon atoms; R₅ to R₆ represent a hydrogen atom or a monovalent organic group having 1 to 20 carbon atoms, which optionally contains a cyclic ether structure having 2 to 13 carbon atoms; R₇ to R₁₀ represent a hydrogen atom or a monovalent organic group having 1 to 20 carbon atoms, which optionally contains a cyclic ether structure having 2 to 13 carbon atoms; R₁₁ represents a monovalent organic group having 1 to 20 carbon atoms, which optionally contains a cyclic ether structure having 2 to 13 carbon atoms; and Y represents a divalent organic group having 1 to 10 carbon atoms; wherein compounds represented by the above general formulae (1-A) to (1-D) contain at least one or more cyclic ether structures having 2 to 13 carbon atoms; in the above general formula (1-A), R₂ and R₃ optionally bond together to form a cyclic ether structure having 2 to 13 carbon atoms; in the above general formula (1-B), adjacent R₅ and R₆ optionally bond together to form an unsaturated or saturated ring structure; and in the above general formula (1-C), adjacent R₈ and R₉ optionally bond together to form a cyclic ether structure having 2 to 13 carbon atoms or an unsaturated or saturated ring structure.

When the compound for forming a metal-containing film that contains the multidentate ligand having such structure is used in a composition for forming a resist underlayer film, higher adhesiveness to a resist upper layer film can be obtained, thereby further effective in preventing collapse of a fine pattern.

In this case, the multidentate ligand preferably has the structure represented by the above formula (1-A) or (1-B).

Such multidentate ligand can provide the compound for forming a metal-containing film having excellent stability.

Furthermore, the cyclic ether structure is preferably represented by any of the following formulae (a-1) and (a-2), 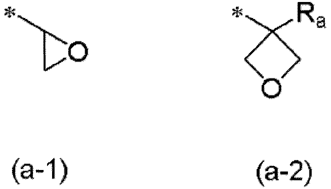 wherein Rₐ represents a hydrogen atom or an organic group having 1 to 10 carbon atoms; and * represents a bonding site.

When such cyclic ether structure is contained, an epoxy or oxetane structure causes a ring opening reaction during film formation by heating to cure a film. Additionally, a hydroxyl group generated by this ring opening reaction also contributes to enhancing adhesiveness to a resist upper layer film.

Furthermore, the compound for forming a metal-containing film is preferably a reaction product of a metal-containing compound containing any of a metal compound represented by the following formula (2) and a hydrolyzate, a condensate, or a hydrolysis condensate of the metal compound represented by the following formula (2), and the multidentate ligand having the structure represented by the above general formulae (1-A) to (1-D),

LₐMX_{b} (2)

wherein M represents any of Ti, Zr, and Hf; L represents a monodentate and/or multidentate ligand having 1 to 30 carbon atoms; X represents a hydrolyzable group selected from a halogen atom, an alkoxy group, a carboxylate group, an acyloxy group, and -NR^{a}R^{b}, wherein each of R^{a} and R^{b} independently represents a hydrogen atom or a monovalent organic group having 1 to 20 carbon atoms, satisfying a+b=4 wherein "a" and "b" represent an integer of 0 to 4.

Use of such compound for forming a metal-containing film enables formation of a metal-containing film having excellent dry etching resistance to an oxygen gas.

In this case, the metal compound represented by the formula (2) preferably has a structure represented by the following formula (3),

M(OR^{1A})₄ (3)

wherein M represents Ti, Zr, or Hf; and R^{1A} represents a monovalent organic group having 1 to 20 carbon atoms.

The compound for forming a metal-containing film having such structure is preferable from the viewpoints of productivity and availability of raw materials.

Furthermore, the compound for forming a metal-containing film preferably further contains a ligand derived from a silicon compound represented by the following general formula (4), 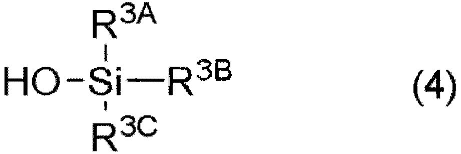 in the above general formula (4), R^{3A}, R^{3B}, and R^{3C} represent any organic group selected from an organic group having 1 to 30 carbon atoms, having a crosslinking group with a structure represented by any of the following general formulae (b-1) to (b-3), a substituted or unsubstituted alkyl group having 1 to 20 carbon atoms and aryl group having 1 to 20 carbon atoms; 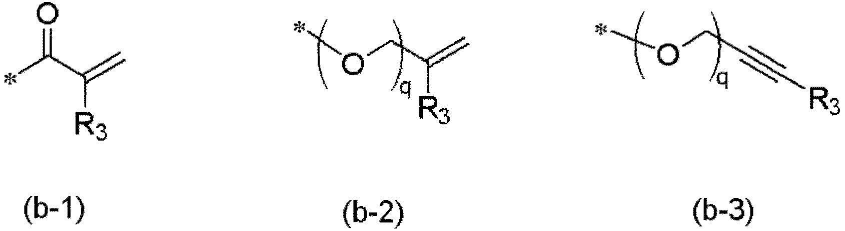 in the above general formulae (b-1) to (b-3), R₃ represents a hydrogen atom or a monovalent organic group having 1 to 10 carbon atoms; "q" represents 0 or 1; and * represents a bonding site.

By containing such ligand, it is possible to further enhance the stability of the compound for forming a metal-containing film.

The present invention also provides a composition for forming a metal-containing film, containing:
(A) the compound for forming a metal-containing film; and
(B) an organic solvent.

Such composition for forming a metal-containing film enables formation of a metal-containing film having high adhesiveness to a resist upper layer film and an effect of preventing collapse of a fine pattern, as well as enabling to obtain a favorable pattern shape. Furthermore, the metal-containing film thus obtained shows a high refractive index such that it can achieve a high antireflective effect in ArF immersion/under High-NA exposure conditions.

Furthermore, the composition may contain one or more of (C) a thermal acid generator and (D) a photoacid generator.

The composition for forming a metal-containing film containing the above additives will achieve more excellent application performance and adhesiveness to a resist upper layer film.

Furthermore, the composition may further contain one or more of (E) a crosslinking agent and (F) a surfactant.

The composition for forming a metal-containing film containing the above additives will achieve more excellent application performance, dry etching resistance, and filling and/or planarization properties.

Furthermore, (B) the organic solvent is preferably a mixture of one or more kinds of organic solvents having a boiling point of less than 180°C and one or more kinds of organic solvents having a boiling point of 180°C or higher.

By adding a high boiling point solvent to impart flowability to the compound for forming a metal-containing film, it is possible to prevent application defects from occurring due to dryness of the composition for forming a metal-containing film.

The present invention also provides a patterning process for forming a pattern in a substrate to be processed, including the steps of:
(I-1) applying the composition for forming a metal-containing film on a substrate to be processed and thereafter performing heat treatment to form a metal-containing film;
(I-2) forming a resist upper layer film on the metal-containing film using a photoresist material;
(I-3) forming a pattern in the resist upper layer film by developing the resist upper layer film using a developer after pattern exposure;
(I-4) transferring the pattern to the metal-containing film by dry etching while using the resist upper layer film having the formed pattern as a mask; and
(I-5) processing the substrate to be processed while using the metal-containing film having the formed pattern as a mask to form the pattern in the substrate to be processed.

The present invention also provides a patterning process for forming a pattern in a substrate to be processed, including the steps of:
(II-1) forming a resist underlayer film on a substrate to be processed;
(II-2) applying the composition for forming a metal-containing film on the resist underlayer film and
thereafter performing heat treatment to form a metal-containing film;
(II-3) forming a resist upper layer film on the metal-containing film using a photoresist material;
(II-4) forming a pattern in the resist upper layer film by developing the resist upper layer film using a developer after pattern exposure;
(II-5) transferring the pattern to the metal-containing film by dry etching while using the resist upper layer film having the formed pattern as a mask;
(II-6) transferring the pattern to the resist underlayer film by dry etching while using the metal-containing film having the formed pattern as a mask; and
(II-7) processing the substrate to be processed while using the resist underlayer film having the formed pattern as a mask to form the pattern in the substrate to be processed.

Accordingly, the compound for forming a metal-containing film of the present invention contains, for example, the cyclic ether structure selected from the above formula (a-1) or (a-2), and when used in the composition for forming a metal-containing film, this compound enables formation of a metal-containing film with excellent adhesiveness to a resist upper layer film. Therefore, it can be suitably used in various patterning processes such as a two-layer resist process and a three-layer resist process. These patterning processes can effectively prevent pattern collapse by utilizing the metal-containing film, so that they are suitable for photolithography of the resist upper layer film and enables highly accurate transfer of a resist upper layer film pattern to the substrate to be processed.

### ADVANTAGEOUS EFFECTS OF INVENTION

As described above, the present invention provides: a compound for forming a metal-containing film having high adhesiveness to a resist upper layer film and an effect of preventing collapse of a fine pattern; a composition for forming a metal-containing film using the compound; and a patterning process using the composition.

Since a resist underlayer film formed using a conventional compound for forming a metal-containing film has much higher surface energy (or a smaller contact angle) than that of a subsequently applied photoresist, such mismatch of the surface energy causes poor adhesion between the metal-containing film and the subsequently applied photoresist, resulting in pattern collapse. Furthermore, when an organic polymer is mixed as a surface modifier, dry etching resistance becomes insufficient during processing of an organic underlayer film, and there is a risk of causing poor transfer of a pattern.

In contrast, the compound for forming a metal-containing film of the present invention contains a cyclic ether structure, so that using it for the composition for forming a metal-containing film enables formation of a metal-containing film having excellent adhesiveness to a resist upper layer film. Therefore, it has an effect of preventing collapse of a fine pattern and provides a substrate to be processed with a pattern shape of the resist upper layer film having high rectangularity, thereby extremely useful in a multilayer resist process. Additionally, the compound for forming a metal-containing film using the multidentate ligand of the present invention enables formation of a resist underlayer film having a higher proportion of a metal atom as compared to a compound for forming a metal-containing film using a monodentate ligand. Therefore, it has excellent resistance against dry etching using an oxygen gas and a fluorine gas respectively, enabling formation of a fine pattern in a substrate to be processed with high precision in various patterning processes such as a two-layer resist process and a three-layer resist process.

### BRIEF DESCRIPTION OF DRAWINGS

FIG. 1 is a diagram illustrating one example of the patterning process (three-layer resist process) according to the present invention;
FIG. 2 is a diagram illustrating one example of the patterning process (two-layer resist process) according to the present invention; and
FIG. 3 is a diagram illustrating calculation of a reflectance in the patterning process.

### DESCRIPTION OF EMBODIMENTS

As described above, it has been demanded to develop: a compound for forming a metal-containing film that yields a metal-containing film enabling to obtain a favorable pattern shape and having high adhesiveness to a resist upper layer film to prevent collapse of a fine pattern in a fine patterning process in a semiconductor device manufacturing process; a composition for forming a metal-containing film using the compound; and a patterning process using the composition.

The present inventors have made an intensive investigation on the above problems, and consequently found that the above problems can be solved by a compound for forming a metal-containing film containing a ligand having a specific structure, a composition for forming a metal-containing film using the compound, and a patterning process using the composition, and completed the present invention.

That is, the present invention provides a compound for forming a metal-containing film, containing: at least one metal atom selected from a group consisting of Ti, Zr, and Hf; and a multidentate ligand coordinated to the metal atom and containing a cyclic ether structure having 2 to 13 carbon atoms.

Hereinafter, the present invention will be described in detail. However, the present invention is not limited thereto.

### <Compound for Forming Metal-Containing Film>

The present invention provides a compound for forming a metal-containing film usable in a composition for forming a metal-containing film, the compound containing: at least one metal atom selected from a group consisting of Ti, Zr, and Hf; and a multidentate ligand coordinated to the metal atom and containing a cyclic ether structure having 2 to 13 carbon atoms.

By using such compound for forming a metal-containing film in a composition for forming a resist underlayer film, it is possible to form a metal-containing film having high adhesiveness to a resist upper layer film and an effect of preventing collapse of a fine pattern, as well as enabling to obtain a favorable pattern shape. Furthermore, the metal-containing film thus obtained shows a high refractive index such that it can achieve a high antireflective effect in ArF immersion/under High-NA exposure conditions.

The multidentate ligand preferably has a structure represented by any of the following general formulae (1-A) to (1-D), 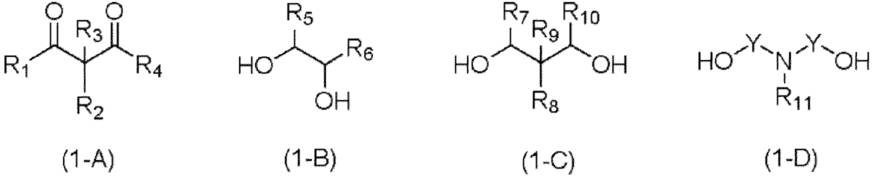 in the above general formulae (1-A) to (1-D), R₁ to R₄ represent a hydrogen atom or a monovalent organic group having 1 to 20 carbon atoms, which optionally contains a cyclic ether structure having 2 to 13 carbon atoms; R₅ to R₆ represent a hydrogen atom or a monovalent organic group having 1 to 20 carbon atoms, which optionally contains a cyclic ether structure having 2 to 13 carbon atoms; R₇ to R₁₀ represent a hydrogen atom or a monovalent organic group having 1 to 20 carbon atoms, which optionally contains a cyclic ether structure having 2 to 13 carbon atoms; R₁₁ represents a monovalent organic group having 1 to 20 carbon atoms, which optionally contains a cyclic ether structure having 2 to 13 carbon atoms; and Y represents a divalent organic group having 1 to 10 carbon atoms; wherein compounds represented by the above general formulae (1-A) to (1-D) contain at least one or more cyclic ether structures having 2 to 13 carbon atoms; in the above general formula (1-A), R₂ and R₃ optionally bond together to form a cyclic ether structure having 2 to 13 carbon atoms; in the above general formula (1-B), adjacent R₅ and R₆ optionally bond together to form an unsaturated or saturated ring structure, and in the above general formula (1-C), adjacent R₈ and R₉ optionally bond together to form a cyclic ether structure having 2 to 13 carbon atoms or an unsaturated or saturated ring structure.

The compounds represented by the above formulae (1-A) to (1-D) contain at least one or more cyclic ether structures having 2 to 13 carbon atoms, preferably 2 to 10 carbon atoms. More preferably the one or two cyclic ether structures, and further preferably the one cyclic structure is contained in a molecule.

In the above general formula (1-A), R₁ and R₄ represent a hydrogen atom or a monovalent organic group having 1 to 20 carbon atoms, which optionally contains a cyclic ether structure having 2 to 13 carbon atoms; preferably, a hydrogen atom, a substituted or unsubstituted alkyl group having 1 to 10 carbon atoms, a substituted or unsubstituted aryl group having 6 to 20 carbon atoms, a substituted or unsubstituted allyl group having 2 to 10 carbon atoms, a substituted or unsubstituted ethynyl group having 2 to 10 carbon atoms, or a monovalent organic group having 1 to 20 carbon atoms, containing a cyclic ether structure having 2 to 13 carbon atoms; and more preferably, a methyl group, an ethyl group, a phenyl group, an allyloxy group, a propargyloxy group, an alkoxy group having 1 to 10 carbon atoms, or a monovalent organic group having 1 to 20 carbon atoms, containing a cyclic ether structure having 2 to 13 carbon atoms. When R₁ and R₄ do not contain a cyclic ether structure having 2 to 13 carbon atoms, they are preferably a methyl group, a methoxy group, or an ethoxy group.

R₂ and R₃ represent a hydrogen atom or a monovalent organic group having 1 to 20 carbon atoms, which optionally contains a cyclic ether structure having 2 to 13 carbon atoms; preferably, a hydrogen atom, a substituted or unsubstituted alkyl group having 1 to 10 carbon atoms, a substituted or unsubstituted aryl group having 6 to 20 carbon atoms, a substituted or unsubstituted allyl group having 2 to 10 carbon atoms, a substituted or unsubstituted ethynyl group having 2 to 10 carbon atoms, or a monovalent organic group having 1 to 20 carbon atoms, containing a cyclic ether structure having 2 to 13 carbon atoms; and more preferably, a hydrogen atom, a methyl group, an ethyl group, a phenyl group, an allyloxy group, a propargyloxy group, or a monovalent organic group having 1 to 20 carbon atoms, containing a cyclic ether structure having 2 to 13 carbon atoms. Additionally, R₂ and R₃ optionally bond together to form a cyclic ether structure having 2 to 13 carbon atoms. When R₂ has a substituent other than a hydrogen atom, R₃ is preferably a hydrogen atom. At least one of R₂ and R₃ is preferably a hydrogen atom. When R₂ and R₃ do not contain a cyclic ether structure having 2 to 13 carbon atoms, both of R₂ and R₃ are preferably a hydrogen atom.

In the above general formula (1-B), R₅ and R₆ represent a hydrogen atom or a monovalent organic group having 1 to 20 carbon atoms, which optionally contains a cyclic ether structure having 2 to 13 carbon atoms; and preferably, a hydrogen atom, a substituted or unsubstituted alkyl group having 1 to 10 carbon atoms, a substituted or unsubstituted aryl group having 6 to 20 carbon atoms, a substituted or unsubstituted allyl group having 2 to 10 carbon atoms, a substituted or unsubstituted ethynyl group having 2 to 10 carbon atoms, or a monovalent organic group having 1 to 20 carbon atoms, containing a cyclic ether structure having 2 to 13 carbon atoms. When R₅ and R₆ do not contain a cyclic ether structure having 2 to 13 carbon atoms, they are preferably a hydrogen atom. R₅ and R₆ optionally bond together to form an unsaturated or saturated ring structure.

In the above formula (1-C), R₇ and R₁₀ represent a hydrogen atom or a monovalent organic group having 1 to 20 carbon atoms, which optionally contains a cyclic ether structure having 2 to 13 carbon atoms; preferably, a hydrogen atom or a substituted or unsubstituted alkyl group having 1 to 10 carbon atoms; and more preferably, a hydrogen atom.

In the above formula (1-C), R₈ represents a hydrogen atom or a monovalent organic group having 1 to 20 carbon atoms, which optionally contains a cyclic ether structure having 2 to 13 carbon atoms, and more preferably a monovalent organic group having 1 to 20 carbon atoms, containing a cyclic ether structure having 2 to 13 carbon atoms. R₉ represents a hydrogen atom or a monovalent organic group having 1 to 20 carbon atoms, which optionally contains a cyclic ether structure having 2 to 13 carbon atoms; more preferably, a hydrogen atom, or an alkyl group or an aryl group having 1 to 10 carbon atoms; and further preferably, a hydrogen atom. Additionally, R₈ and R₉ optionally bond together to form a cyclic ether structure having 2 to 13 carbon atoms or an unsaturated or saturated ring structure.

In the above formula (1-D), R₁₁ preferably represents a divalent organic group having 1 to 20 carbon atoms, containing a cyclic ether structure having 2 to 13 carbon atoms. Y represents a divalent organic group having 1 to 10 carbon atoms, preferably a divalent hydrocarbon group having 1 to 10 carbon atoms, more preferably a hydrocarbon group having 1 to 5 carbon atoms from the viewpoint of availability of raw materials, further preferably a methylene group or an ethylene group, and particularly preferably an ethylene group.

When used in a composition for forming a resist underlayer film, the compound for forming a metal-containing film containing such multidentate ligand can achieve higher adhesiveness to a resist upper layer film, thereby further effective in preventing collapse of a fine pattern.

The cyclic ether structure (cyclic ether structure having 2 to 13 carbon atoms) is preferably represented by any of the following formulae (a-1) and (a-2), 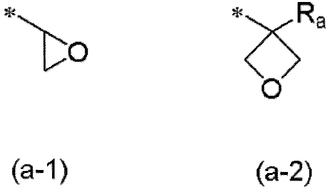 wherein Rₐ represents a hydrogen atom or an organic group having 1 to 10 carbon atoms; and * represents a bonding site.

In such compound for forming a metal-containing film, an epoxy or oxetane structure causes a ring opening reaction during film formation by heating to cure a film. Additionally, a hydroxyl group generated by this ring opening reaction also contributes to enhancing adhesiveness to a resist upper layer film.

Rₐ in the above (a-2) preferably represents a hydrogen atom, a methyl group, or an ethyl group, and more preferably an ethyl group.

Examples of a structure of the ligand derived from the formulae (1-A) to (1-D) include the following compounds, but the present invention is not limited thereto. 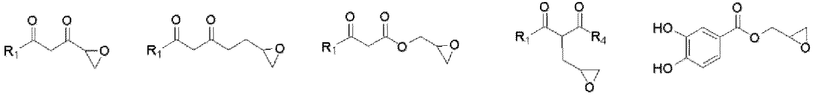 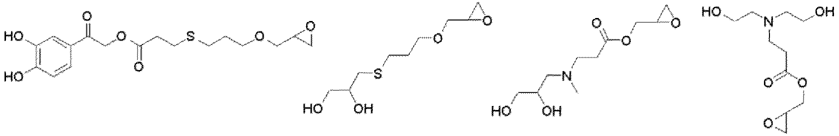 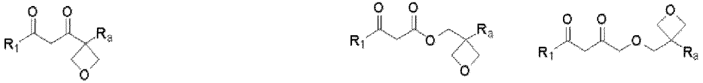 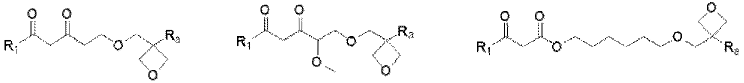 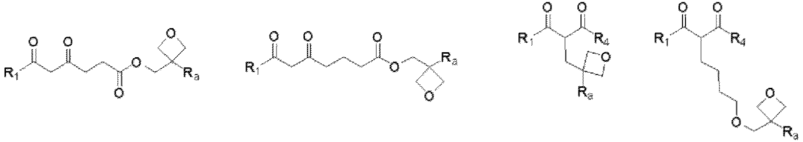 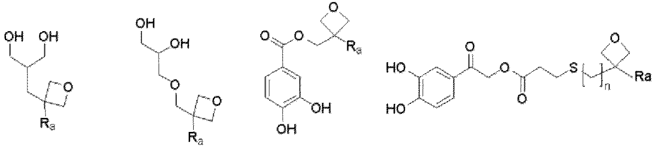 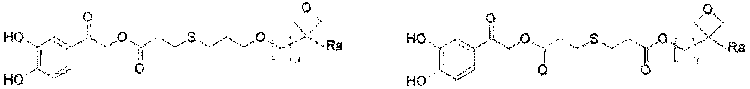 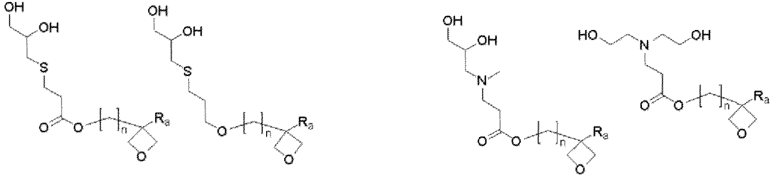 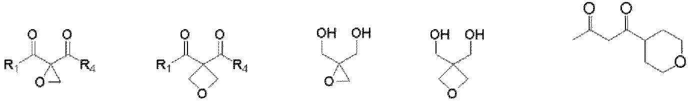 (Wherein R₁, R₄, and Rₐ are the same as the above; and "n" represents 1 to 10.)

From the viewpoints of productivity and stability of the compound for forming a metal-containing film, the cyclic ether structure is more preferably the structure represented by the formula (a-2).

The multidentate ligand more preferably has the structure represented by the above formula (1-A) or (1-B) .

Such multidentate ligand can provide the compound for forming a metal-containing film having excellent stability.

Furthermore, the compound for forming a metal-containing film is preferably a reaction product of (a) the metal-containing compound containing any of a metal compound represented by the following formula (2) and a hydrolyzate, a condensate, or a hydrolysis condensate of the metal compound represented by the following formula (2), and the multidentate ligand having the structure represented by the above general formulae (1-A) to (1-D),

LₐMX_{b} (2)

wherein M represents any of Ti, Zr, and Hf; L represents a monodentate and/or multidentate ligand having 1 to 30 carbon atoms; X represents a hydrolyzable group selected from a halogen atom, an alkoxy group, a carboxylate group, an acyloxy group, and -NR^{a}R^{b}, wherein each of R^{a} and R^{b} independently represents a hydrogen atom or a monovalent organic group having 1 to 20 carbon atoms, satisfying a+b=4 wherein "a" and "b" represent an integer of 0 to 4.

When used in a composition for forming a metal-containing film, such compound for forming a metal-containing film enables formation of a metal-containing film with excellent dry etching resistance to an oxygen gas.

### (a) Metal-Containing Compound

### (Hydrolyzable Group)

Examples of the hydrolyzable group X in the above formula (2) include halogen atom, alkoxy group, carboxylate group, acyloxy group, and -NR^{a}R^{b}. Preferably, each of R^{a} and R^{b} independently represents a hydrogen atom or a monovalent organic group having 1 to 20 carbon atoms.

Examples of the above halogen atom include fluorine atom, chlorine atom, bromine atom, iodine atom, and the like.

Examples of the above alkoxy group include methoxy group, ethoxy group, n-propoxy group, i-propoxy group, n-butoxy group, t-butoxy group, and the like.

The above carboxylate group includes acetate group, propionate group, butyrate group, n-hexanecarboxylate group, n-octanecarboxylate group, and the like.

Examples of the above acyloxy group include acetoxy group, ethyryloxy group, propionyloxy group, butyryloxy group, t-butyryloxy group, t-amyryloxy group, n-hexanecarbonyloxy group, n-octanecarbonyloxy group, and the like.

Examples of the above -NR^{a}R^{b} include unsubstituted amino group, methylamino group, dimethylamino group, diethylamino group, dipropylamino group, and the like.

As the above hydrolyzable group X, an alkoxy group is preferable, and an i-propoxy group, an n-butoxy group, or a t-butoxy group is more preferable.

### (Monodentate Ligand)

Examples of the monodentate ligand L in the above formula (2) include hydroxo ligand, carboxy ligand, amide ligand, amine ligand, ammonia ligand, olefin ligand, and the like.

Examples of the above amide ligand include unsubstituted amide ligand (NH₂), methylamide ligand (NHMe), dimethylamide ligand (NMe₂), diethylamide ligand (NEt₂), dipropylamide ligand (NPr₂), and the like.

Examples of the above amine ligand include pyridine, trimethylamine ligand, piperidine ligand, and the like.

Examples of the olefine ligand include: chain olefine such as ethylene and propylene; cyclic olefine such as cyclopentene, cyclohexene, and norbornene; and the like.

### (Multidentate Ligand)

Examples of the multidentate ligand L in the above formula (2) include a ligand derived from hydroxy acid ester, a ligand derived from β-diketone, a ligand derived from β-ketoester, a ligand derived from α,α-dicarboxylate ester, a hydrocarbon having a π bond, diphosphine, and the like.

Examples of the above hydroxy acid ester include glycolate ester, lactic acid ester, 2-hydroxycyclohexane-1-carboxylate ester, salicylate ester, and the like.

Examples of the above β-diketone include acetoacetic ester, α-alkyl-substituted acetoacetic ester, β-ketopentanoic acid ester, benzoylacetic acid ester, 1,3-acetonedicarboxylate ester, and the like.

Examples of the above α,α-dicarboxylate ester include malonic acid diester, α-alkyl-substituted malonic acid diester, α-cycloalkyl-substituted malonic acid diester, α-aryl-substituted malonic acid diester, and the like.

Examples of the above hydrocarbon having a π bond include: chain dienes such as butadiene and isoprene; cyclic dienes such as cyclopentadiene, methylcyclopentadiene, pentamethylcyclopentadiene, cyclohexadiene, and norbornadiene; aromatic hydrocarbons such as benzene, toluene, xylene, hexamethylbenzene, naphthalene, and indene; and the like.

Examples of the above diphosphine include 1,1-bis(diphenylphosphino)methane, 1,2-bis(diphenylphosphino)ethane, 1,3-bis(diphenylphosphino)propane, 2,2'-bis(diphenylphosphino)-1,1'-binaphthyl, 1,1'-bis(diphenylphosphino)ferrocene, and the like.

In the above general formula (2), the relation of a+b=4 is satisfied, wherein "a" and "b" represent an integer of 0 to 4. "a" is preferably 0 to 3, more preferably 0 to 2, further preferably 1 to 2, and particularly preferably 2. "b" is preferably 2 to 4, more preferably 2 or 3, and further preferably 2. By setting "a" and "b" in the above ranges, it is possible to enhance stability of (A) the metal compound.

Examples of (a) the metal-containing compound include: compounds containing titanium such as diisopropoxybis(2,4-pentanedionato)titanium(IV), tetra-n-butoxytitanium(IV), tetra-n-propoxytitanium(IV), tri-n-butoxymonostearate titanium(IV), titanium(IV)butoxide oligomer, aminopropyl trimethoxy titanium(IV), triethoxy mono(2,4-pentanedionato)titanium(IV), tri-n-propoxy mono(2,4-pentanedionato)titanium(IV), triisopropoxy mono(2,4-pentanedionato)titanium, and di-n-butoxybis(2,4-pentanedionato)titanium(IV); compounds containing zirconium such as dibutoxybis(ethyl acetoacetate)zirconium(IV), di-n-butoxybis(2,4-pentanedionato)zirconium(IV), tetra-n-butoxyzirconium(IV), tetra-n-propoxyzirconium(IV), tetraisopropoxyzirconium(IV), aminopropyltriethoxyzirconium(IV), 2-(3,4-epoxycyclohexyl)ethyltrimethoxyzirconium(IV), γ-glycidoxypropyltrimethoxyzirconium(IV), 3-isocyanopropyltrimethoxyzirconium(IV), triethoxy mono(2,4-pentanedionato)zirconium(IV), tri-n-propoxy mono(2,4-pentanedionato)zirconium(IV), triisopropoxy mono(2,4-pentanedionato)zirconium(IV), tri(3-methacryloxypropyl)methoxyzirconium(IV), and tri(3-acryloxypropyl)methoxyzirconium(IV); and compounds containing hafnium such as diisopropoxybis(2,4-pentanedionato)hafnium(IV), tetrabutoxyhafnium(IV), tetraisopropoxyhafnium(IV), tetraethoxyhafnium(IV), and dichlorobis(cyclopentadienyl)hafnium(IV).

From the view point of availability of raw materials, the metal compound represented by the formula (2) preferably has a structure represented by the following formula (3),

M(OR^{1A})₄ (3)

wherein M represents Ti, Zr, or Hf; and R^{1A} represents a monovalent organic group having 1 to 20 carbon atoms.

In the formula (3), R^{1A} represents a monovalent organic group having 1 to 20 carbon atoms, preferably a methyl group, an ethyl group, an n-propyl group, an i-propyl group, an n-butyl group, or a t-butyl group, and more preferably an isopropyl group, an n-butyl group, or a t-butyl group.

### (b) Compound for Forming Ligand

During a synthesis reaction of the compound for forming a metal-containing film, a compound (hereinafter referred to as (b) compound for forming a ligand) that can become a monodentate or multidentate ligand in the compound for forming a metal-containing film may be added, in addition to (a) the metal-containing compound and the multidentate ligand containing the cyclic ether structure having 2 to 13 carbon atoms.

The above (b) compound for forming a ligand includes: organic compounds derived from a hydroxo ligand, a carboxy ligand, an amide ligand, an amine ligand, an ammonia ligand, an olefin ligand, and the like listed as L in the above formula (2); organic compounds derived from a ligand derived from hydroxy acid ester, a ligand derived from β-diketone, a ligand derived from β-ketoester, a ligand derived from α,α-dicarboxylate ester, and the like; a compound having a plurality of hydroxy groups; and the like.

Furthermore, (b) the compound for forming a ligand may be an organic compound having 1 to 30 carbon atoms, containing at least one or more crosslinking groups represented by any of the following general formulae (c-1) to (c-4), (d-1) to (d-4), and (e-1) to (e-3). 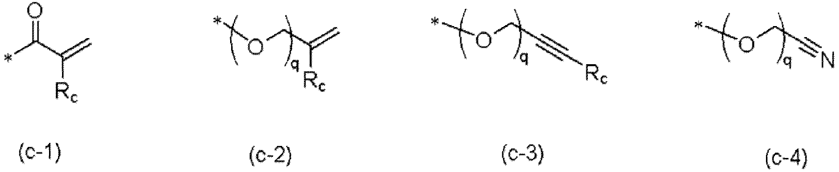 (In the above general formulae (c-1) to (c-4), R_{c} represents a hydrogen atom or a monovalent organic group having 1 to 10 carbon atoms; "q" represents 0 or 1; and * represents a bonding site.) 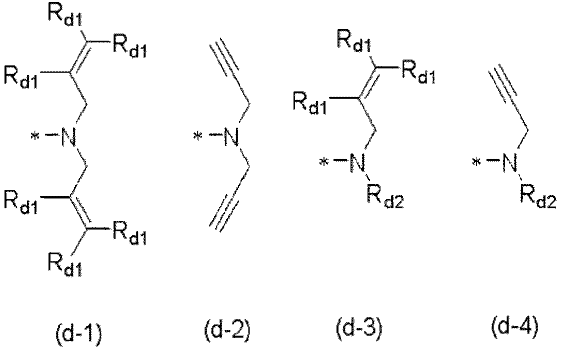 (In the above general formulae, each R_{d1} represents a hydrogen atom or a methyl group, which may be the same or different from each other in the same formula; R_{d2} represents a hydrogen atom, a substituted or unsubstituted, saturated or unsaturated monovalent organic group having 1 to 20 carbon atoms, a substituted or unsubstituted aryl group having 6 to 30 carbon atoms, or a substituted or unsubstituted arylalkyl group having 7 to 31 carbon atoms; and * represents a bonding site.) 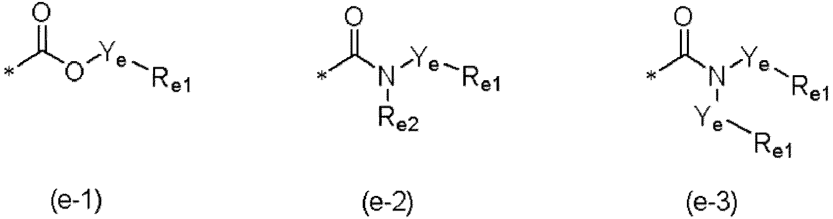 (In the above general formulae (e-1) to (e-3), Yₑ represents a divalent organic group having 1 to 31 carbon atoms, preferably 1 to 20 carbon atoms; Rₑ₂ represents a hydrogen atom, a substituted or unsubstituted saturated monovalent organic group having 1 to 20 carbon atoms or unsaturated monovalent organic group having 2 to 20 carbon atoms, a substituted or unsubstituted aryl group having 6 to 30 carbon atoms, or a substituted or unsubstituted arylalkyl group having 7 to 31 carbon atoms; Rₑ₁ represents an organic group represented by the following general formula (e-4), where a protective group leaves due to an action of either or both of acid and heat to generate one or more hydroxyl groups or carboxyl groups; and * represents a bonding site.) 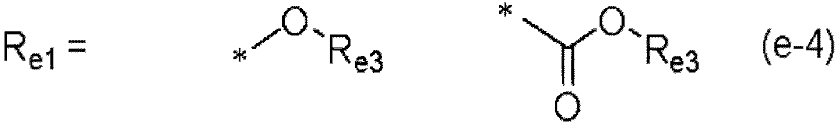 (In the above general formula (e-4), Rₑ₃ represents an organic group in which a protective group leaves due to an action of either or both of acid and heat; and * represents a bonding site to Yₑ.)

When (b) the compound for forming a ligand contains at least one or more crosslinking groups represented by any of the above (c-1) to (c-4), (d-1) to (d-4), and (e-1) to (e-3), sufficient thermal flowability can be obtained during film formation and thus, by using it for the composition for forming a metal-containing film, it is possible to form a metal-containing film with excellent flatness.

From the viewpoints of productivity and stability of the compound for forming a metal-containing film, (b) the compound for forming a ligand is more preferably a compound derived from a carboxy ligand, a compound derived from a ligand derived from β-diketone, and a compound having a plurality of hydroxy groups. These can be used alone or in combination of two or more thereof.

Examples of the compound derived from a carboxy ligand include a compound represented by the following formula (5), 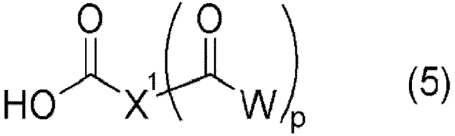 in the above general formula (5), "p" represents 0 or 1; when "p" is 1, X¹ represents a divalent organic group having 2 to 20 carbon atoms, and W represents any of an alkoxy group having 1 to 10 carbon atoms, the following general formula (5-A), and the above general formulae (d-1) to (d-4); when "p" is 0, X¹ represents a monovalent organic group having 8 to 30 carbon atoms, containing an aromatic ring and the crosslinking group having any of the structures represented by the above general formulae (c-2) to (c-4); 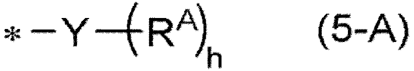 in the above general formula (5-A), Y represents a saturated divalent organic group having 1 to 20 carbon atoms or unsaturated divalent organic group having 2 to 20 carbon atoms; R^{A} represents any of the structures represented by the above general formulae (c-1) to (c-4); "h" represents 1 to 6; and * represents a bonding site to a carbon atom of carbonyl.

The compound derived from a carboxy ligand having such structure can enhance a thermosetting property of the compound for forming a metal-containing film, enabling formation of a metal-containing film with excellent resistance against mixing with a resist upper layer film.

Preferably in the general formula (5), "p" represents 1 and W represents a structure represented by the following general formula (5-B), 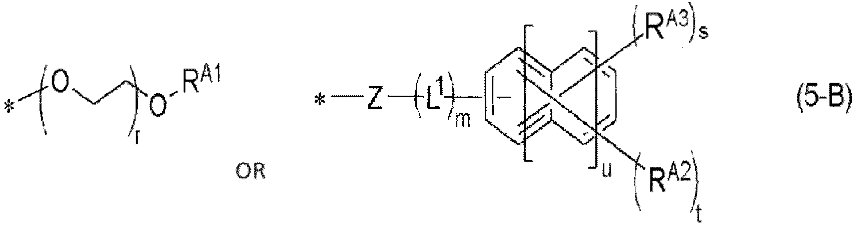 in the above general formula (5-B), R^{A1} represents the structure represented by the above general formula (c-1); R^{A2} represents any of the structures represented by the above general formula (c-2) or (c-3); Z represents either an oxygen atom or a secondary amine; L¹ represents a divalent hydrocarbon group having 1 to 10 carbon atoms; R^{A3} represents a saturated monovalent organic group having 1 to 20 carbon atoms or unsaturated monovalent organic group having 2 to 20 carbon atoms; "t" represents 1 to 6, "s" represents 0 to 5, and t+s is 1 or greater and 6 or less; "r" represents 1 to 10; "u" represents 0 or 1; "m" represents 0 or 1; and * represents a bonding site to a carbon atom of carbonyl.

The compound derived from a carboxy ligand having such structure can enhance a heat resistance property of the compound for forming a metal-containing film so that a formed metal-containing film exhibits excellent film formability, and further reduce generation of a sublimate during heat curing, thereby preventing contamination of an apparatus.

Preferably in the general formula (5), "p" represents 1; W represents an alkoxy group having 1 to 10 carbon atoms; and X¹ represents a structure represented by any of the following general formulae (5-C) . 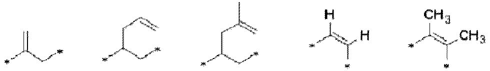 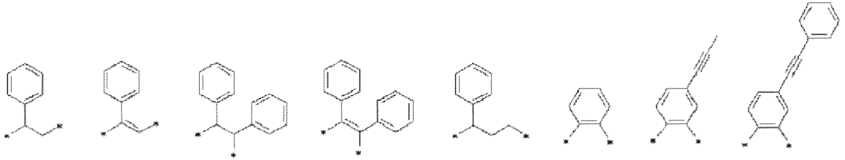 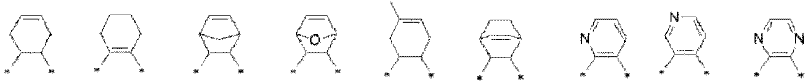 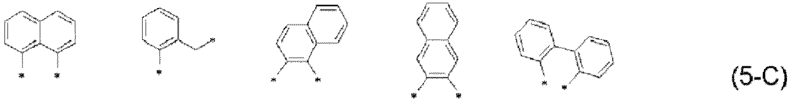 (Wherein * represents a bonding site.)

The compound derived from a carboxy ligand having such structure can enhance the heat resistance property of the compound for forming a metal-containing film so that a formed metal-containing film exhibits excellent film formability, and further reduce generation of a sublimate during heat curing, thereby preventing contamination of an apparatus.

Alternatively, in the general formula (5), it is preferable that "p" represents 0 and X¹ represents a structure represented by any of the following general formulae (5-D). 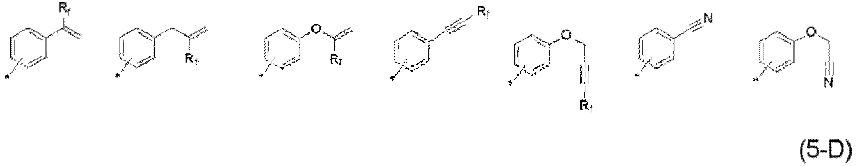 (In the above general formulae (5-D), R_{f} is the same as R_{c} in the above general formula (c-2) or (c-3); and * represents a bonding site to a carbon atom of carbonyl.)

Examples of the compound derived from a carboxy ligand include a compound represented by the following formula (6), 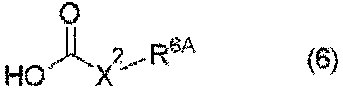 in the above general formula (6), X² represents a divalent organic group having 1 to 31 carbon atoms; and R^{6A} represents any of the above general formulae (e-1) to (e-3) .

In the above general formula (6), X² represents a divalent organic group having 1 to 31 carbon atoms, and more preferably a saturated hydrocarbon having 1 to 20 carbon atoms or unsaturated hydrocarbon having 2 to 20 carbon atoms from the viewpoint of thermosetting.

In the above general formulae (e-1) to (e-3), Yₑ represents a substituted or unsubstituted saturated divalent organic group having 1 to 20 carbon atoms or unsaturated divalent organic group having 2 to 20 carbon atoms, a substituted or unsubstituted aryl group having 6 to 30 carbon atoms, or a substituted or unsubstituted arylalkyl group having 7 to 31 carbon atoms; and preferably, Yₑ represents a saturated hydrocarbon having 1 to 20 carbon atoms or unsaturated hydrocarbon having 2 to 20 carbon atoms.

In the general formulae (e-1) to (e-3), when Yₑ represents a saturated hydrocarbon having 1 to 20 carbon atoms or unsaturated hydrocarbon having 2 to 20 carbon atoms, it is possible to further enhance the thermosetting property of the compound for forming a metal-containing film. Additionally, it is preferable from the viewpoint of availability of raw materials.

Specific examples of Yₑ include, but are not limited to, the following structures, 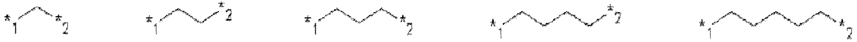 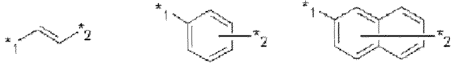 wherein *₁ represents a bonding site to an oxygen atom or a nitrogen atom; and *₂ represents a bonding site to Rₑ₁.

In the above general formula (e-4), Rₑ₃ represents an organic group in which a protective group (thermolabile and acid labile group) leaves due to an action of either or both of acid and heat, preferably a tertiary hydrocarbyl group or a group that forms, together with an adjacent oxygen atom, an acetal structure, and particularly preferably a tertiary hydrocarbyl group. The tertiary hydrocarbyl group has preferably 4 to 20 carbon atoms, and more preferably smaller number of carbon atoms within the range.

Examples of the compound derived from a ligand derived from β-diketone include a compound represented by a formula (7), 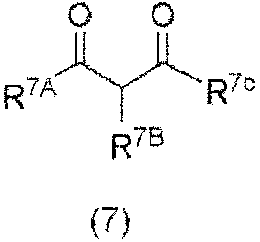 in the above general formula, R^{7A} to R^{7c} represent a hydrogen atom or a monovalent organic group having 1 to 20 carbon atoms, which optionally contains the crosslinking group represented by any of the above general formulae (c-1) to (c-4) and (d-1) to (d-4).

The compound represented by the above general formula (7) preferably contains at least one or more crosslinking groups represented by any of the above general formulae (c-1) to (c-4) and (d-1) to (d-4). Such compound is preferable because it can further enhance the thermosetting property of the compound for forming a metal-containing film.

Examples of the compound having a plurality of hydroxy groups include a dihydric alcohol represented by any of the formulae (7-A) to (7-C), and the like, 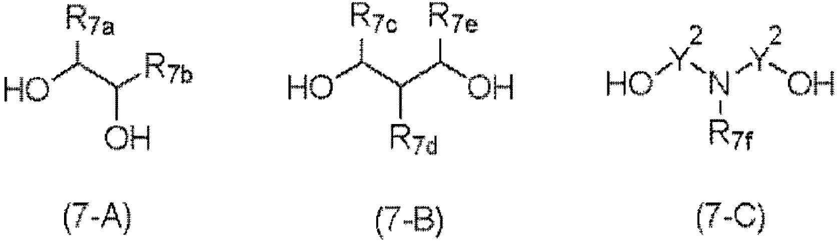 in the above general formulae, R₇ₐ to R_{7b} represent a hydrogen atom or a monovalent organic group having 1 to 20 carbon atoms, which optionally contains the crosslinking group represented by any of the above general formulae (c-1) to (c-4); R_{7c} to R_{7f} represent a hydrogen atom or a monovalent organic group having 1 to 20 carbon atoms, which optionally contains the crosslinking group represented by any of the general formulae (c-1) to (c-4) and (d-1) to (d-4); Y² represents a divalent organic group having 1 to 10 carbon atoms; and in the above general formula (7-A), adjacent R₇ₐ and R_{7b} optionally bond together to form a unsaturated or saturated ring structure.

The compounds represented by the above general formulae (7-A) to (7-C) preferably contain at least one or more crosslinking groups represented by any of the above general formulae (c-1) to (c-4) and (d-1) to (d-4). Such compounds are preferable because they can further enhance the thermosetting property of the compound for forming a metal-containing film.

The content of the multidentate ligand containing the cyclic ether structure having 2 to 13 carbon atoms in the compound for forming a metal-containing film is preferably 10 mole % to 100 mole %, more preferably 20 mole % to 90 mole %, and further preferably 25 mole % to 75 mole % of the total of the ligands coordinated to the metal atom. The content of the ligand derived from (b) the compound for forming a ligand is preferably 0 mole % to 50 mole % and more preferably 0 mole % to 25 mole % of the total of the ligands coordinated to the metal atom. The content of a ligand derived from other than (b) the compound for forming a ligand, for example, a ligand derived from an alkoxy group having 1 to 10 carbon atoms, is preferably 0 mole % to 90 mole % and more preferably 0 mole % to 75 mole % of the total of the ligands coordinated to the metal atom.

### (c) Silicon-Containing Compound

Furthermore, during the synthesis reaction of the compound for forming a metal-containing film, (c) a silicon-containing compound may be added in addition to the multidentate ligand containing the cyclic ether structure having 2 to 13 carbon atoms.

By substituting the hydrolyzable group of (a) the metal-containing compound with the silicon-containing compound, it is possible to enhance the stability of the compound for forming a metal-containing film in the composition for forming a metal-containing film.

Examples of the silicon-containing compound include a structure represented by the following general formula (4), and the like, 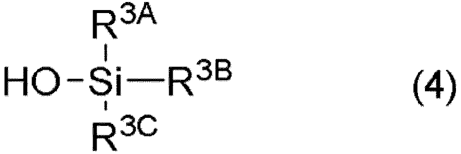 in the above general formula (4), R^{3A}, R^{3B}, and R^{3C} represent any organic group selected from an organic group having 1 to 30 carbon atoms, having a crosslinking group with a structure represented by any of the following general formulae (b-1) to (b-3), a substituted or unsubstituted alkyl group having 1 to 20 carbon atoms and aryl group having 1 to 20 carbon atoms; 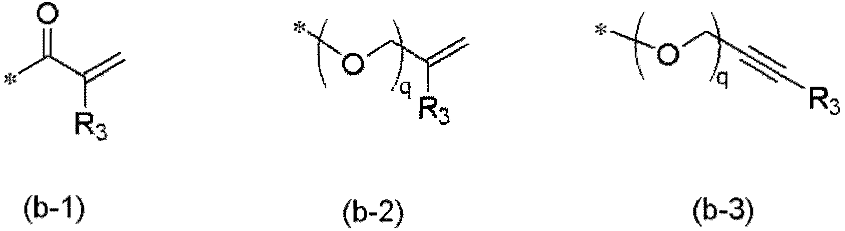 in the above general formulae (b-1) to (b-3), R₃ represents a hydrogen atom or a monovalent organic group having 1 to 10 carbon atoms; "q" represents 0 or 1; and * represents a bonding site.

As the silicon-containing compound, any groups represented by the following formulae are preferable, and trimethylsilanol is more preferable from the viewpoint of productivity, 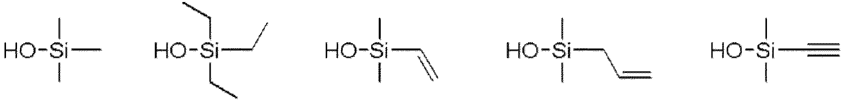 wherein * represents a bonding site to a metal atom.

When the compound for forming a metal-containing film contains the multidentate ligand containing the cyclic ether structure having 2 to 13 carbon atoms and a ligand derived from (c) the silicon-containing compound, the content of the multidentate ligand containing the cyclic ether structure having 2 to 13 carbon atoms in the compound for forming a metal-containing film is preferably 10 mole % to 100 mole %, more preferably 20 mole % to 80 mole %, and further preferably 25 mole % to 75 mole % of the total of the ligands coordinated to the metal atom. The content of the ligand derived from (c) the silicon-containing compound is preferably 10 mole % to 90 mole %, more preferably 20 mole % to 80 mole %, and further preferably 25 mole % to 75 mole % of the total of the ligands coordinated to the metal atom. The content of the multidentate ligand containing the cyclic ether structure having 2 to 13 carbon atoms and a ligand derived from other than (c) the silicon-containing compound, for example, a ligand derived from an alkoxy group having 1 to 10 carbon atoms, is preferably 0 mole % to 90 mole % and more preferably 0 mole % to 75 mole % of the total of the ligands coordinated to the metal atom.

### Synthesis Method

When synthesizing the compound for forming a metal-containing film of the present invention, the synthesis method is not particularly limited as long as the compound for forming a metal-containing film contains the multidentate ligand containing the cyclic ether structure having 2 to 13 carbon atoms. For example, metal alkoxide or metal acetylacetonate (acac) can be used in (a) the metal-containing compound, such that the compound for forming a metal-containing film can be obtained by reacting an alkoxy or acac metal with the multidentate ligand containing the cyclic ether structure having 2 to 13 carbon atoms. (a) the metal-containing compound may be subjected to hydrolysis condensation, followed by a reaction with the multidentate ligand containing the cyclic ether structure having 2 to 13 carbon atoms, or (a) the metal-containing compound may be reacted with the multidentate ligand containing the cyclic ether structure having 2 to 13 carbon atoms, followed by the hydrolysis condensation. If it is difficult to control the hydrolysis condensation, it may be reacted with the multidentate ligand containing the cyclic ether structure having 2 to 13 carbon atoms in a non-water environment. These are preferably adjusted as appropriate according to properties required for the compound for forming a metal-containing film and the metal-containing film. When (c) the silicon-containing compound and the multidentate ligand containing the cyclic ether structure having 2 to 13 carbon atoms as a ligand are contained, it is preferable that (a) the metal-containing compound is reacted with (c) the silicon-containing compound, and thereafter reacted with the multidentate ligand containing the cyclic ether structure having 2 to 13 carbon atoms.

Examples of a method for carrying out a hydrolysis condensation reaction using (a) the metal-containing compound include a method for carrying out the hydrolysis condensation reaction of (a) the metal-containing compound in a solvent containing water, etc. In this case, other compounds having a hydrolyzable group may be added as necessary. Additionally, an acid such as acetic acid may be added as a catalyst for the hydrolysis condensation reaction. The lower limit of the amount of the water used in this hydrolysis condensation reaction is preferably 0.2 times, more preferably 1 times, and further preferably 3 times the mole of the hydrolyzable group contained in (a) the metal-containing compound or the like. The upper limit of the amount of the water is preferably 20 times, more preferably 15 times, and further preferably 10 times by mole.

A solvent used for the synthesis reaction of the compound for forming a metal-containing film is not particularly limited, and the same solvent as those exemplified as (B) an organic solvent described later can be used, for example. A typical solvent and solvent mixture contain ester, ether, or an alcohol functional group, that is a 70:30 by volume mixture of propylene glycol monomethyl ether acetate (PGMEA) and propylene glycol monomethyl ether (PGME), for example. Other examples of the usable solvent include butanediol monomethyl ether, ethylene glycol monomethyl ether, butanediol monoethyl ether, propylene glycol monoethyl ether, ethylene glycol monoethyl ether, butanediol monopropyl ether, propylene glycol monopropyl ether, ethylene glycol monopropyl ether, ethylene glycol monobutyl ether, diethylene glycol monomethyl ether, diethylene glycol monoethyl ether, diethylene glycol monopropyl ether, diethylene glycol monobutyl ether, propylene glycol monobutyl ether, 1-butanol, 2-butanol, 2-methyl-1-propanol, 4-methyl-2-pentanol, acetone, tetrahydrofuran, toluene, hexane, ethyl acetate, cyclohexanone, methyl amyl ketone, propylene glycol dimethyl ether, diethylene glycol dimethyl ether, diamyl ether, isoamyl ether, propylene glycol monomethyl ether acetate, propylene glycol monoethyl ether acetate, ethyl pyruvate, butyl acetate, methyl 3-methoxypropionate, ethyl 3-ethoxypropionate, t-butyl acetate, t-butyl propionate, propylene glycol mono-t-butyl ether acetate, γ-butyrolactone, methylisobutyl ketone, cyclopentyl methyl ether, and the like.

### <Composition for Forming Metal-Containing Film>

The present invention also provides a composition for forming a metal-containing film, containing (A) the compound for forming a metal-containing film of the present invention; and (B) an organic solvent.

### <(A) Compound for Forming Metal-Containing Film>

The component (A) in the composition for forming a metal-containing film of the present invention is the aforementioned compound for forming a metal-containing film of the present invention. The blending amount of the component (A) in the composition is not particularly limited, and can be, for example, 1 to 20 parts by mass, preferably 1 to 10 parts by mass, and more preferably 1 to 5 parts by mass relative to 100 parts by mass of the total amount of the composition.

### <(B) Organic Solvent>

(B) the organic solvent usable in the composition for forming a metal-containing film of the present invention is not particularly limited as long as it is capable of dissolving (A) the compound for forming a metal-containing film described above, as well as (C) a thermal acid generator, (D) a photoacid generator, (E) a crosslinking agent, (F) a surfactant, and other additives (if contained) described later.

Specifically, for example, organic solvents described in paragraphs [0091] to [0092] of JP 2007-199653 A can be added. More specifically, propylene glycol monomethyl ether acetate, propylene glycol monomethyl ether, propylene glycol monoethyl ether, propylene glycol monopropyl ether, 2-heptanone, cyclopentanone, cyclohexanone, γ-butyrolactone, or a mixture of one or more thereof is preferably used.

The blending amount of the organic solvent is preferably in the range of 200 to 10,000 parts and more preferably 250 to 5,000 parts relative to 100 parts by mass of (A) the compound for forming a metal-containing film.

### <High Boiling Point Solvent>

In the composition for forming a metal-containing film of the present invention, (B) the organic solvent may contain a high boiling point organic solvent. The high boiling point organic solvent can be one or more kinds of organic solvents having a boiling point of 180 degrees (°C) or higher.

For example, used as (B) the organic solvent may be a mixture of one or more kinds of organic solvents having a boiling point of less than 180°C and one or more kinds of organic solvents having a boiling point of 180°C or higher (high boiling point solvent).

The high boiling point solvent is not particularly limited to hydrocarbons, alcohols, ketones, esters, ethers, chlorinated solvents, or the like, as long as it is capable of dissolving each component of the composition for forming a metal-containing film of the present invention. Specific examples thereof include 1-octanol, 2-ethylhexanol, 1-nonanol, 1-decanol, 1-undecanol, ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, glycerin, n-nonyl acetate, monohexyl ether, ethylene glycol mono-2-ethylhexyl ether, ethylene glycol monophenyl ether, ethylene glycol monobenzyl ether, diethylene glycol monoethyl ether, diethylene glycol monoisopropyl ether, diethylene glycol mono-n-butyl ether, diethylene glycol monoisobutyl ether, diethylene glycol monohexyl ether, diethylene glycol monophenyl ether, diethylene glycol monobenzyl ether, diethylene glycol diethyl ether, diethylene glycol dibutyl ether, diethylene glycol butyl methyl ether, triethylene glycol dimethyl ether, triethylene glycol monomethyl ether, triethylene glycol-n-butyl ether, triethylene glycol butyl methyl ether, tetraethylene glycol dimethyl ether, dipropylene glycol monomethyl ether, dipropylene glycol mono-n-propyl ether, dipropylene glycol mono-n-butyl ether, tripropylene glycol dimethyl ether, tripropylene glycol monomethyl ether, tripropylene glycol mono-n-propyl ether, tripropylene glycol mono-n-butyl ether, ethylene glycol monoethyl ether acetate, ethylene glycol monobutyl ether acetate, diethylene glycol monomethyl ether acetate, diethylene glycol monoethyl ether acetate, diethylene glycol monobutyl ether acetate, triacetin, propylene glycol diacetate, dipropylene glycol methyl-n-propyl ether, dipropylene glycol methyl ether acetate, 1,4-butanediol diacetate, 1,3-butylene glycol diacetate, 1,6-hexanediol diacetate, triethylene glycol diacetate, γ-butyrolactone, methyl benzoate, ethyl benzoate, propyl benzoate, butyl benzoate, dihexyl malonate, diethyl succinate, dipropyl succinate, dibutyl succinate, dihexyl succinate, dimethyl adipate, diethyl adipate, dibutyl adipate, and the like. These may be used alone or in mixture thereof.

The high boiling point solvent may be appropriately selected from the ones described above, for example, according to a temperature at which the composition for forming a metal-containing film of the present invention is subjected to heat treatment. The boiling point of the high boiling point solvent is preferably 180°C to 300°C and further preferably 200°C to 300°C. At such boiling point, there is no risk of evaporation progressing too quickly during baking (heat treatment), so that it is possible to prevent defects due to dryness from occurring during film formation. Furthermore, at such boiling point, the high boiling point solvent never remains in a film without evaporating after baking, so that there is no risk of adversely affecting physical properties of the film such as etching resistance.

Additionally, the blending amount of the high boiling point solvent (if used) is preferably 1 to 30 parts by mass relative to 100 parts by mass of the organic solvent having a boiling point of less than 180°C. Such blending amount is preferable because it can impart sufficient thermal flowability during baking and the high boiling point solvent does not remain in a film, causing no deterioration in physical properties of the film such as etching resistance.

### <Other Components>

When the composition for forming a metal-containing film is a composition for forming a metal-containing film usable as a resist underlayer film used in a multilayer resist method, it contains one or more (A) the compound for forming a metal-containing film and (B) the organic solvent, and may contain at least one or more of (C) a thermal acid generator, (D) a photoacid generator, (E) a crosslinking agent, and (F) a surfactant as necessary.

Hereinafter, components other than (A) the compound for forming a metal-containing film and (B) the organic solvent will be described, that can be contained in the composition for forming a metal-containing film of the present invention.

### (C) Thermal Acid Generator

(C) the thermal acid generator is preferably added to the composition for forming a metal-containing film of the present invention to facilitate a heat-induced crosslinking reaction.

(C) the thermal acid generator usable in the composition for forming a metal-containing film of the present invention includes the one represented by the following general formula (7) and the like. 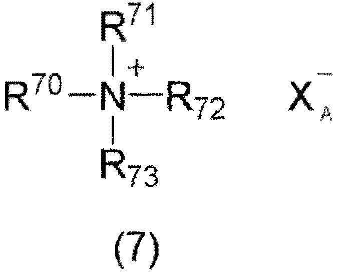 (Wherein X_{A}⁻ represents a non-nucleophilic counter ion; each of R⁷⁰, R⁷¹, R⁷², and R⁷³ represents a hydrogen atom or a linear, branched, or cyclic alkyl group, alkenyl group, oxoalkyl group, or oxoalkenyl group having 1 to 12 carbon atoms, an aryl group having 6 to 20 carbon atoms, or an aralkyl group or aryloxoalkyl group having 7 to 12 carbon atoms, wherein part or all of hydrogen atoms in these groups is optionally substituted with an alkoxy group or the like; additionally, R⁷⁰ and R⁷¹, or R⁷⁰, R⁷¹, and R⁷² optionally form a ring, and when they form a ring, R⁷⁰ and R⁷¹, or R⁷⁰, R⁷¹, and R⁷² represent an alkylene group having 3 to 10 carbon atoms or a heteroaromatic ring having a nitrogen atom in the formula in the ring.

The above R⁷⁰, R⁷¹, R⁷², and R⁷³ may be the same or different from each other, and specific examples of the alkyl group include methyl group, ethyl group, propyl group, isopropyl group, n-butyl group, sec-butyl group, tert-butyl group, pentyl group, hexyl group, heptyl group, octyl group, cyclopentyl group, cyclohexyl group, cycloheptyl group, cyclopropylmethyl group, 4-methylcyclohexyl group, cyclohexylmethyl group, norbornyl group, adamantyl group, and the like.

The alkenyl group includes vinyl group, allyl group, propenyl group, butenyl group, hexenyl group, cyclohexenyl group, and the like.

The oxoalkyl group includes 2-oxocyclopentyl group, 2-oxocyclohexyl group, 2-oxopropyl group, 2-cyclopentyl-2-oxoethyl group, 2-cyclohexyl-2-oxoethyl group, 2-(4-methylcyclohexyl)-2-oxoethyl group, and the like.

The oxoalkenyl group includes 2-oxo-4-cyclohexenyl group, 2-oxo-4-propenyl group, and the like.

The aryl group includes: phenyl group, naphthyl group, and the like; alkoxyphenyl groups such as p-methoxyphenyl group, m-methoxyphenyl group, o-methoxyphenyl group, ethoxyphenyl group, p-tert-butoxyphenyl group, and m-tert-butoxyphenyl group; alkylphenyl groups such as 2-methylphenyl group, 3-methylphenyl group, 4-methylphenyl group, ethylphenyl group, 4-tert-butylphenyl group, 4-butylphenyl group, and dimethylphenyl group; alkylnaphthyl groups such as methylnaphthyl group and ethylnaphthyl group; alkoxynaphthyl groups such as methoxynaphthyl group and ethoxynaphthyl group; dialkylnaphthyl groups such as dimethylnaphthyl group and diethylnaphthyl group; dialkoxynaphthyl groups such as dimethoxynaphthyl group and diethoxynaphthyl group; and the like.

The aralkyl group includes benzyl group, phenylethyl group, phenetyl group, and the like.

The aryloxoalkyl group includes 2-aryl-2-oxoethyl groups such as 2-phenyl-2-oxoethyl group, 2-(1-naphthyl)-2-oxoethyl group, and 2-(2-naphthyl)-2-oxoethyl group.

Furthermore, when R⁷⁰ and R⁷¹, or R⁷⁰, R⁷¹, and R⁷² form a heteroaromatic ring having the nitrogen atom in the formula in the ring, examples thereof include imidazole derivatives (for example, imidazole, 4-methylimidazole, 4-methyl-2-phenylimidazole, and the like), pyrazole derivatives, furazan derivatives, pyrroline derivatives (for example, pyrroline, 2-methyl-1-pyrroline, and the like), pyrrolidine derivatives (for example, pyrrolidine, N-methylpyrrolidine, pyrrolidinone, N-methylpyrrolidone, and the like), imidazoline derivatives, imidazolidine derivatives, pyridine derivatives (for example, pyridine, methylpyridine, ethylpyridine, propylpyridine, butylpyridine, 4-(1-butylpentyl)pyridine, dimethylpyridine, trimethylpyridine, triethylpyridine, phenylpyridine, 3-methyl-2-phenylpyridine, 4-tert-butylpyridine, diphenylpyridine, benzylpyridine, methoxypyridine, butoxypyridine, dimethoxypyridine, 1-methyl-2-pyridone, 4-pyrrolidinopyridine, 1-methyl-4-phenylpyridine, 2-(1-ethylpropyl)pyridine, aminopyridine, dimethylaminopyridine, and the like), pyridazine derivatives, pyrimidine derivatives, pyrazine derivatives, pyrazoline derivatives, pyrazolidine derivatives, piperidine derivatives, piperazine derivatives, morpholine derivatives, indole derivatives, isoindole derivatives, 1H-indazole derivatives, indoline derivatives, quinoline derivatives (for example, quinoline, 3-quinolinecarbonitrile, and the like), isoquinoline derivatives, cinnoline derivatives, quinazoline derivatives, quinoxaline derivatives, phthalazine derivatives, purine derivatives, pteridine derivatives, carbazole derivatives, phenanthridine derivatives, acridine derivatives, phenazine derivatives, 1,10-phenanthroline derivatives, adenine derivatives, adenosine derivatives, guanine derivatives, guanosine derivatives, uracil derivatives, uridine derivatives, and the like.

The aforementioned non-nucleophilic counter ion of X_{A}⁻ includes: halide ions such as chloride ion and bromide ion; fluoroalkylsulfonates such as triflate, 1,1,1-trifluoroethanesulfonate, and nonafluorobutanesulfonate; arylsulfonates such as tosylate, benzenesulfonate, 4-fluorobenzenesulfonate, and 1,2,3,4,5-pentafluorobenzenesulfonate; alkylsulfonates such as mesylate and butanesulfonate; imide acids such as bis(trifluoromethylsulfonyl)imide, bis(perfluoroethylsulfonyl)imide, and bis(perfluorobutylsulfonyl)imide; methide acids such as tris(trifluoromethylsulfonyl)methide and tris(perfluoroethylsulfonyl)methide; furthermore, a sulfonate with an α-position substituted with fluoro, represented by the following general formula (8); a sulfonate with α- and β-positions substituted with fluoro, represented by the following general formula (9); and the like.

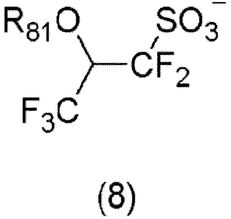

R₉₁CF₂CF₂SO₃ (9)

In the above general formula (8), R⁸¹ represents a hydrogen atom, a linear, branched, or cyclic alkyl group or acyl group having 1 to 23 carbon atoms, an alkenyl group having 2 to 20 carbon atoms, or an aryl group or aryloxy group having 6 to 20 carbon atoms. In the above general formula (9), R⁹¹ represents a hydrogen atom, a linear, branched, or cyclic alkyl group having 1 to 20 carbon atoms, an alkenyl group having 2 to 20 carbon atoms, or an aryl group having 6 to 20 carbon atoms.

Specific examples of the above thermal acid generator include the following, but are not limited thereto.

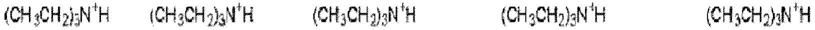

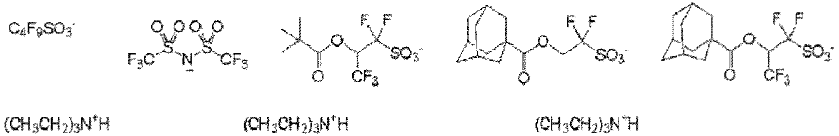

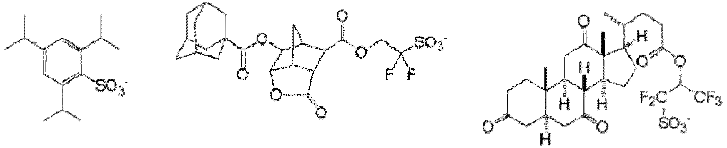

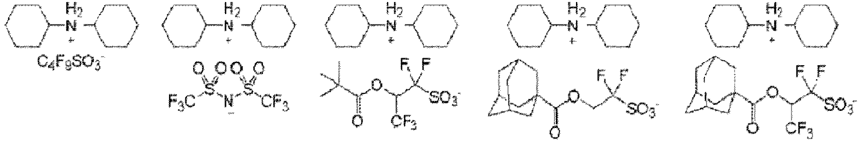

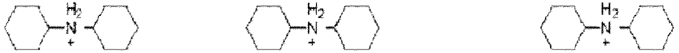

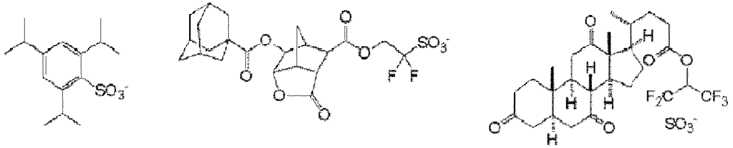

(C) the thermal acid generator contained in the composition for forming a metal-containing film of the present invention can be used alone or in combination of two or more kinds thereof. The addition amount of the thermal acid generator is preferably 0.05 to 30 parts and more preferably 0.1 to 10 parts relative to 100 parts of (A) the compound for forming a metal-containing film. If it is 0.05 parts or more, a sufficient amount of acid generation and a sufficient crosslinking reaction can be obtained, and if it is 30 parts or less, there is less possibility of a mixing phenomenon due to acid migration to an upper layer resist.

### (D) Photoacid Generator

(D) the photoacid generator can be added to the composition for forming a metal-containing film of the present invention to appropriately adjust pattern shape, exposure sensitivity, etc. of a resist upper layer film. The photoacid generator can be used alone or in combination of two or more kinds thereof.

For example, those described in paragraphs [0160] to [0179] of JP 2009-126940 A can be used as the photoacid generator. The addition amount of the photoacid generator is preferably 0.05 to 30 parts and more preferably 0.1 to 10 parts relative to 100 parts of (A) the compound for forming a metal-containing film. If the addition amount of the photoacid generator is within the above range, resolution is good and there is no risk of problems of foreign substances occurring after resist development or during stripping.

### (E) Crosslinking Agent

(E) the crosslinking agent can also be added to the composition for forming a metal-containing film of the present invention to enhance a curing property and further prevent intermixing with a resist upper layer film.

The crosslinking agent is not particularly limited, and various known types of crosslinking agents can widely be used. One example thereof includes: melamine-based crosslinking agent, glycoluril-based crosslinking agent, benzoguanamine-based crosslinking agent, urea-based crosslinking agent, β-hydroxyalkylamide-based crosslinking agent, isocyanurate-based crosslinking agent, aziridine-based crosslinking agent, oxazoline-based crosslinking agent, epoxy-based crosslinking agent, and phenol-based crosslinking agent. (E) the crosslinking agent can be used alone or in combination of two or more kinds thereof, and the addition amount of the crosslinking agent (if added) is preferably 5 to 50 parts and more preferably 10 to 40 parts relative to 100 parts of (A) the compound for forming a metal-containing film. If the addition amount is 5 parts or more, the sufficient curing property can be exhibited so as to prevent intermixing with a resist upper layer film. On the other hand, if the addition amount is 50 parts or less, there is no risk of deterioration in dry etching resistance due to a decreased proportion of (A) the compound for forming a metal-containing film in the composition.

Specific examples of the melamine-based crosslinking agent include hexamethoxymethylated melamine, hexabutoxymethylated melamine, alkoxy- and/or hydroxy-substituted derivatives thereof, and partial self-condensates thereof.

Specific examples of the glycoluril-based crosslinking agent include tetramethoxymethylated glycoluril, tetrabutoxymethylated glycoluril, alkoxy- and/or hydroxy-substituted derivatives thereof, and partial self-condensates thereof.

Specific examples of the benzoguanamine-based crosslinking agent include tetramethoxymethylated benzoguanamine, tetrabutoxymethylated benzoguanamine, alkoxy- and/or hydroxy-substituted derivatives thereof, and partial self-condensates thereof.

Specific examples of the urea-based crosslinking agent include dimethoxymethylated dimethoxyethyleneurea, alkoxy- and/or hydroxy-substituted derivatives thereof, and partial self-condensates thereof.

Specific examples of the β-hydroxyalkylamide-based crosslinking agent include N,N,N',N'-tetra(2-hydroxyethyl)adipic acid amide.

Specific examples of the isocyanurate-based crosslinking agent include triglycidyl isocyanurate and triallyl isocyanurate.

Specific examples of the aziridine-based crosslinking agent include 4,4'-bis(ethyleneiminocarbonylamino)diphenylmethane and 2,2-bishydroxymethylbutanol-tris[3-(1-aziridinyl)propionate].

Specific examples of the oxazoline-based crosslinking agent include 2,2'-isopropylidenebis(4-benzyl-2-oxazoline), 2,2'-isopropylidenebis(4-phenyl-2-oxazoline), 2,2'-isopropylidenebis(4-phenyl-2-oxazoline), 2,2'-methylenebis-4,5-diphenyl-2-oxazoline, 2,2'-methylenebis-4-phenyl-2-oxazoline, 2,2'-methylenebis-4-tert-butyl-2-oxazoline, 2,2'-bis(2-oxazoline), 1,3-phenylenebis(2-oxazoline), 1,4-phenylenebis(2-oxazoline), and 2-isopropenyloxazoline copolymer.

Specific examples of the epoxy-based crosslinking agent include diglycidyl ether, ethylene glycol diglycidyl ether, 1,4-butanediol diglycidyl ether, 1,4-cyclohexanedimethanol diglycidyl ether, poly(glycidyl methacrylate), trimethylolethane triglycidyl ether, trimethylolpropane triglycidyl ether, and pentaerythritol tetraglycidyl ether.

Specific examples of the phenol-based crosslinking agent include a compound represented by the following general formula (10), 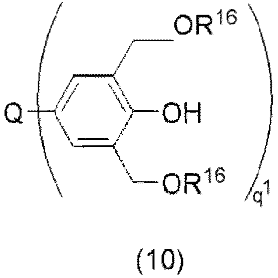 wherein Q represents a single bond or a q¹-valent hydrocarbon group having 1 to 20 carbon atoms; R¹⁶ represents a hydrogen atom or an alkyl group having 1 to 20 carbon atoms; and "q¹" represents an integer of 1 to 5.

Q represents a single bond or a q¹-valent hydrocarbon group having 1 to 20 carbon atoms. "q¹" represents an integer of 1 to 5, and more preferably 2 or 3. Specific examples of Q include methane, ethane, propane, butane, isobutane, pentane, cyclopentane, hexane, cyclohexane, methylpentane, methylcyclohexane, dimethylcyclohexane, trimethylcyclohexane, benzene, toluene, xylene, ethylbenzene, ethylisopropylbenzene, diisopropylbenzene, methylnaphthalene, ethylnaphthalene, and eicosane. R¹⁶ represents a hydrogen atom or an alkyl group having 1 to 20 carbon atoms. Specific examples of the alkyl group having 1 to 20 carbon atoms include methyl group, ethyl group, propyl group, isopropyl group, butyl group, isobutyl group, pentyl group, isopentyl group, hexyl group, octyl group, ethylhexyl group, decyl group, and eicosanyl group. Among them, a hydrogen atom or a methyl group is preferable.

Specific examples of the compound represented by the above general formula (10) include the following compounds. Among them, hexamethoxymethylated forms of triphenolmethane, triphenolethane, 1,1,1-tris(4-hydroxyphenyl)ethane, and tris(4-hydroxyphenyl)-1-ethyl-4-isopropylbenzene are preferable from the viewpoint of enhancing a curing property and film thickness uniformity of an adhesive film. 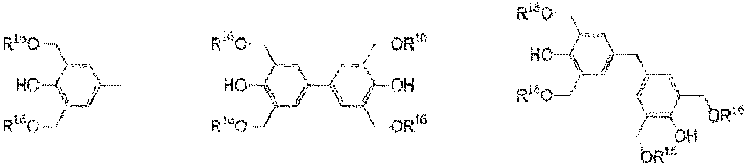 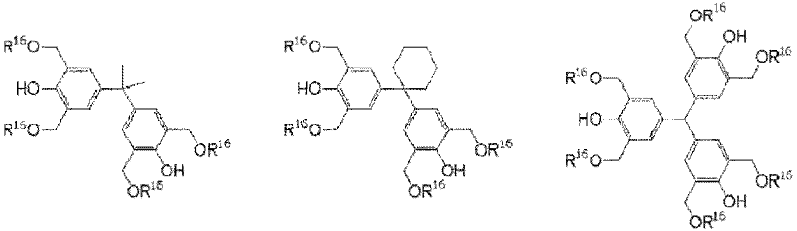 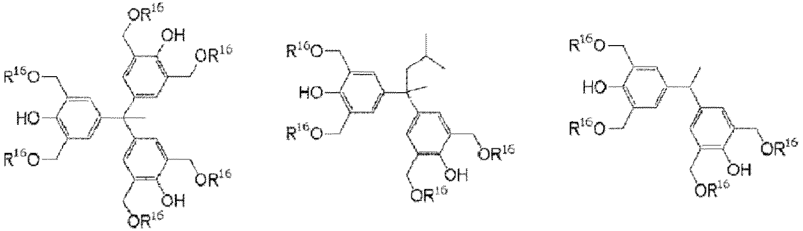 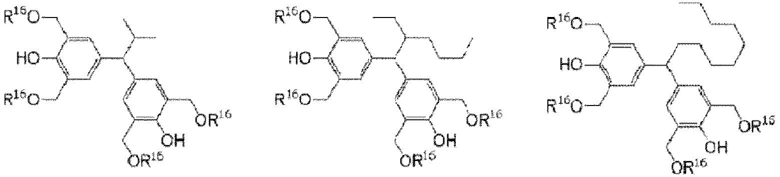 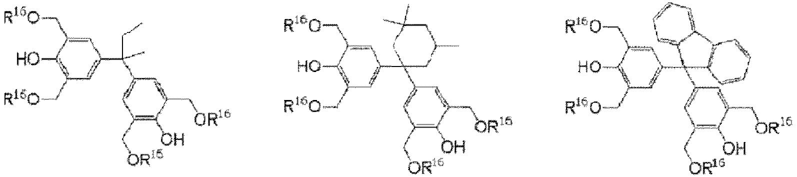 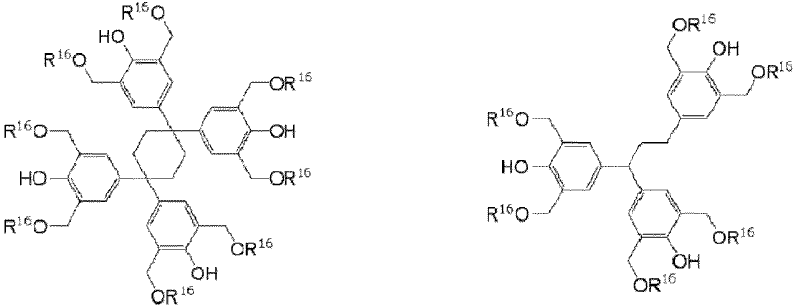 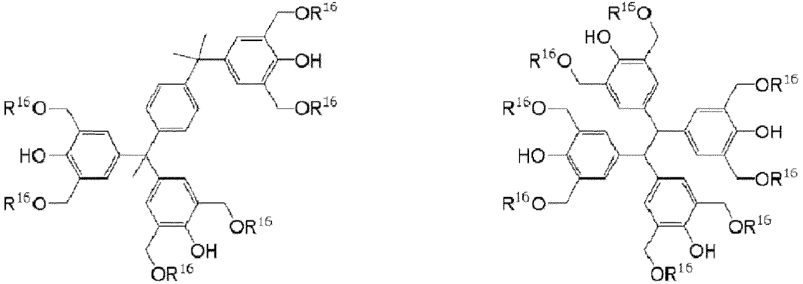 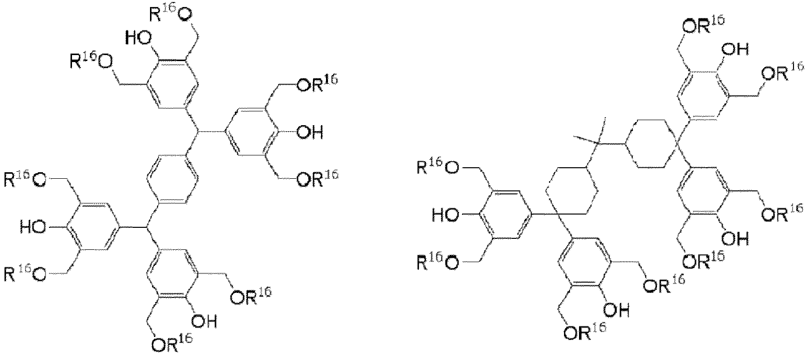 (Wherein R¹⁶ is as described above.)

### (F) Surfactant

(F) the surfactant can be added to the composition for forming a metal-containing film of the present invention to enhance application performance by spin coating. The surfactant can be used alone or in combination of two or more kinds thereof.

For example, those described in paragraphs [0142] to [0147] of JP 2009-269953 A can be used as the surfactant. The addition amount of the surfactant (if added) is preferably 0.001 to 20 parts and more preferably 0.01 to 10 parts relative to 100 parts of (A) the compound for forming a metal-containing film. The addition amount within this range can ensure that the application performance is enhanced to form a thin and uniform adhesive film.

### Plasticizer

A plasticizer can also be added to the composition for forming a metal-containing film of the present invention. The plasticizer is not particularly limited, and various known types of plasticizers can widely be used. One example thereof includes: low molecular weight compounds such as phthalate ester, adipate ester, phosphate ester, trimellitate ester, and citrate ester; and polymers such as polyether-based polymer, polyester-based polymer, and polyacetal-based polymer described in JP 2013-253227 A. The addition amount of the plasticizer (if added) is preferably 1 to 500% by mass relative to 100 parts of (A) the compound for forming a metal-containing film. The addition amount within this range enables excellent filling and levelling of a pattern.

### <Method of Forming Metal-Containing Film>

In the present invention, the composition for forming a metal-containing film as described above can be used to form a metal-containing film as a resist underlayer film of a multilayer resist film used for lithography, for example.

In a method of forming a resist underlayer film using the composition for forming a metal-containing film of the present invention, the composition for forming a metal-containing film is applied on a substrate to be processed by a spin coat method or the like. After the spin coating, baking (heat treatment) is performed to evaporate the solvent and facilitate a crosslinking reaction to prevent intermixing with a resist upper layer film. The baking is preferably performed at 100°C or higher and 450°C or lower for 10 to 600 seconds, more preferably at 200°C or higher and 300°C or lower for 10 to 300 seconds. Considering effects on device damage and deformation of a wafer, the upper limit of a heating temperature in a wafer process of lithography is preferably 450°C or lower and more preferably 300°C or lower.

Furthermore, in the method of forming a resist underlayer film using the composition for forming a metal-containing film of the present invention, a metal-containing film can also be formed by applying the composition for forming a metal-containing film of the present invention on a substrate to be processed by a spin coat method or the like in the same manner as described above, and baking and curing the composition for forming a metal-containing film under an atmosphere with an oxygen concentration of 0.1% by volume or greater and 21% by volume or less.

By baking the composition for forming a metal-containing film of the present invention under such oxygen atmosphere, it is possible to obtain a sufficiently cured film. While the atmosphere during the baking may be air, filling an inert gas such as N₂, Ar, or He to reduce the amount of oxygen is preferable in that the metal-containing film is prevented from oxidation. To prevent oxidation, it is necessary to control the oxygen concentration, which is preferably 1000 ppm or less and more preferably 100 ppm or less (volume basis). It is preferable to prevent oxidation of the metal-containing film during the baking so as not to cause increased absorption or reduced etching resistance.

Alternatively, the composition for forming a metal-containing film can be used to form a metal-containing film as a middle layer film of a multilayer resist film used for lithography, for example.

### <Patterning Process Using Composition for Forming Metal-Containing Film>

As a patterning process with a two-layer resist process using the composition for forming a metal-containing film of the present invention, the present invention also provides a patterning process for forming a pattern in a substrate to be processed, including the steps of:
(I-1) applying the composition for forming a metal-containing film of the present invention on a substrate to be processed and thereafter performing heat treatment to form a metal-containing film;
(I-2) forming a resist upper layer film on the metal-containing film using a photoresist material;
(I-3) forming a pattern in the resist upper layer film by developing the resist upper layer film using a developer after pattern exposure;
(I-4) transferring the pattern to the metal-containing film by dry etching while using the resist upper layer film having the formed pattern as a mask; and
(I-5) processing the substrate to be processed while using the metal-containing film having the formed pattern as a mask to form the pattern in the substrate to be processed.

Since the resist upper layer film in the above two-layer resist process has etching resistance to a chlorine-based gas, dry etching of the metal-containing film in the two-layer resist process while using the resist upper layer film as a mask is preferably performed using an etching gas mainly composed of the chlorine-based gas. Since the metal-containing film has etching resistance to a fluorine-based gas, dry etching of the substrate to be processed while using the metal-containing film as a mask is preferably performed using an etching gas mainly composed of the fluorine-based gas.

As a patterning process with a three-layer resist process using the composition for forming a metal-containing film of the present invention, the present invention also provides a patterning process for forming a pattern in a substrate to be processed, including the steps of:
(II-1) forming a resist underlayer film on a substrate to be processed;
(II-2) applying the composition for forming a metal-containing film of the present invention on the resist underlayer film and thereafter performing heat treatment to form a metal-containing film;
(II-3) forming a resist upper layer film on the metal-containing film using a photoresist material;
(II-4) forming a pattern in the resist upper layer film by developing the resist upper layer film using a developer after pattern exposure;
(II-5) transferring the pattern to the metal-containing film by dry etching while using the resist upper layer film having the formed pattern as a mask;
(II-6) transferring the pattern to the resist underlayer film by dry etching while using the metal-containing film having the formed pattern as a mask; and
(II-7) processing the substrate to be processed while using the resist underlayer film having the formed pattern as a mask to form the pattern in the substrate to be processed.

The metal-containing film formed with the composition for forming a metal-containing film of the present invention, which is used as a middle layer film in the above three-layer resist process, has etching resistance to an oxygen-based gas. Accordingly, in the three-layer resist process, the dry etching of the organic resist underlayer film while using the metal-containing film as a mask is preferably performed by using an etching gas mainly composed of the oxygen-based gas. Since the organic underlayer film has etching resistance to a fluorine-based gas, dry etching of the substrate to be processed while using the organic underlayer film as a mask is preferably performed by using an etching gas mainly composed of the fluorine-based gas.

In the above patterning process, the resist upper layer film may be either a positive or negative type, and the same one as a typically used photoresist composition can be used therefor. In a case of forming the resist upper layer film with the above photoresist composition, the spin coat method is preferable.

When the resist upper layer film is formed by the spin coat method using the photoresist composition, prebaking is performed after resist application, preferably at 60 to 180°C for 10 to 300 seconds. Thereafter, exposure is performed according to a usual manner, followed by post-exposure bake (PEB) and development, thereby obtaining a resist pattern. Note that thickness of the resist upper layer film is not particularly limited but preferably 10 to 500 nm and particularly preferably 20 to 400 nm.

Note that exposure light includes high-energy ray having a wavelength of 300 nm or less; specifically, far ultraviolet ray, KrF excimer laser beam (248 nm), ArF excimer laser beam (193 nm), F₂ laser beam (157 nm), Kr₂ laser beam (146 nm), Ar₂ laser beam (126 nm), soft X-ray (EUV) with 3 to 20 nm, electron beam (EB), ion beam, X-ray, and the like.

The patterning process for the resist upper layer film is preferably a patterning process using photolithography with a wavelength of 5 nm or more and 300 nm or less, direct drawing using an electron beam, nanoimprinting, or a combination thereof.

Additionally, a development method in the patterning process is preferably alkaline development or development using an organic solvent.

Next, etching is performed using the resist pattern thus obtained as a mask. For example, etching of the metal-containing film in the two-layer resist process is performed using a chlorine-based gas while using the upper layer resist pattern as the mask. In this manner, a metal-containing film pattern is formed. Next, etching processing is performed on the substrate to be processed while using the metal-containing film pattern thus obtained as a mask. The etching of a workpiece can be performed according to a usual manner, and when the workpiece is SiO₂, SiN, or a silica-based low dielectric constant insulating film, for example, etching is performed with mainly a fluorocarbon-based gas.

The metal-containing film obtained with the composition for forming a metal-containing film of the present invention is characterized by excellent etching resistance during etching of these workpieces.

Examples of the organic resist underlayer film material usable for the aforementioned resist underlayer film include already known underlayer films for a three-layer resist method or a two-layer resist method using a silicon resist composition. The examples thereof include resins and compositions described in JP 2012-1687 A, JP 2012-77295 A, JP 2004-264710 A, JP 2005-043471 A, JP 2005-250434 A, JP 2007-293294 A, JP 2008-65303 A, JP 2004-205685 A, JP 2007-171895 A, JP 2009-14816 A, JP 2007-199653 A, JP 2008-274250 A, JP 2010-122656 A, JP 2012-214720 A, JP 2014-29435 A, WO 2012/077640 A1, WO 2010/147155 A1, WO 2012/077640 A1, WO 2010/147155 A1, WO 2012/176767 A1, JP 2005-128509 A, JP 2006-259249 A, JP 2006-259482 A, JP 2006-293298 A, JP 2007-316282 A, JP 2012-145897 A, JP 2017-119671 A, JP 2019-44022 A, etc.

The resist underlayer film can be formed on the substrate to be processed by the spin coat method or the like as with the photoresist composition, using, for example, a composition solution containing the above organic resist underlayer film material. After forming the organic resist underlayer film by the spin coat method or the like, baking is preferably performed for evaporating the organic solvent. Preferably, the baking temperature is within a range of 100 to 600°C and baking time is within a range of 10 to 300 seconds.

Instead of the above organic resist underlayer film material, an organic hard mask formed by a CVD or ALD method can also be applied.

In a case of the three-layer resist process, dry etching of the metal-containing film is performed, for example, using a chlorine-based gas while using the upper layer resist pattern as a mask. In this manner, a metal-containing film pattern is formed. Next, etching is performed on the resist underlayer film using an oxygen-based gas while using the metal-containing film pattern thus obtained as a mask. In this manner, a resist underlayer film pattern is formed. Then, the substrate to be processed is processed by etching while using the organic resist underlayer film pattern thus obtained as a mask.

The metal-containing film obtained with the composition for forming a metal-containing film of the present invention is characterized by excellent etching resistance during etching of these resist underlayer films.

Note that the workpiece (substrate to be processed) is not particularly limited, and a substrate made of Si, α-Si, p-Si, SiO₂, SiN, SiON, W, TiN, Al, or the like, the substrate on which a layer to be processed is deposited, etc. are used. As the layer to be processed, various Low-k films such as those made of Si, SiO₂, SiON, SiN, p-Si, α-Si, W, W-Si, Al, Cu, Al-Si, or the like, and a stopper film thereof are used, which can be formed to have thickness of typically 50 to 10,000 nm and particularly 100 to 5,000 nm. Note that in a case of depositing the layer to be processed, the substrate and the layer to be processed for use are made of different materials.

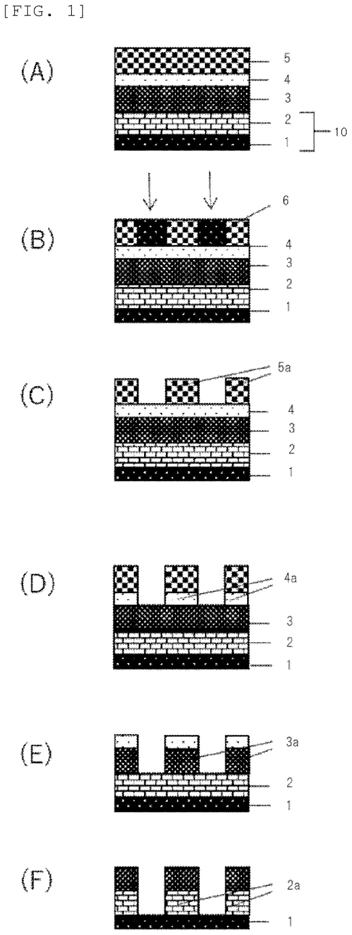

An example of the patterning process with the above three-layer resist process will be described herein with reference to FIG. 1. In this example, firstly, an organic resist underlayer film 3 is formed on a layer to be processed 2 of a substrate to be processed 10 using an organic resist underlayer film material, the substrate to be processed 10 consisting of a substrate 1 and the layer to be processed 2 stacked thereon; a metal-containing film 4 is formed on the organic resist underlayer film 3 using the composition for forming a metal-containing film of the present invention; and a resist upper layer film 5 is formed on the metal-containing film 4 using a photoresist material, as illustrated in FIG. 1(A). Then, an exposed portion 6 of the resist upper layer film is subjected to pattern exposure as illustrated in FIG. 1(B). Next, by development using a developer, a resist upper layer film pattern 5a is formed in the resist upper layer film as illustrated in FIG. 1(C). Next, the pattern is transferred to the metal-containing film 4 by dry etching while using the resist upper layer film having the formed pattern 5a as a mask to obtain a metal-containing film pattern 4a as illustrated in FIG. 1(D). Next, the pattern is transferred to the organic resist underlayer film by dry etching while using the metal-containing film having the formed pattern 4a as a mask to obtain an organic resist underlayer film pattern 3a as illustrated in FIG. 1(E). Then, the layer to be processed 2 on the substrate 1 is processed while using the organic resist underlayer film having the formed pattern 3a as a mask to form a pattern 2a in the substrate to be processed 10 as illustrated in FIG. 1(F).

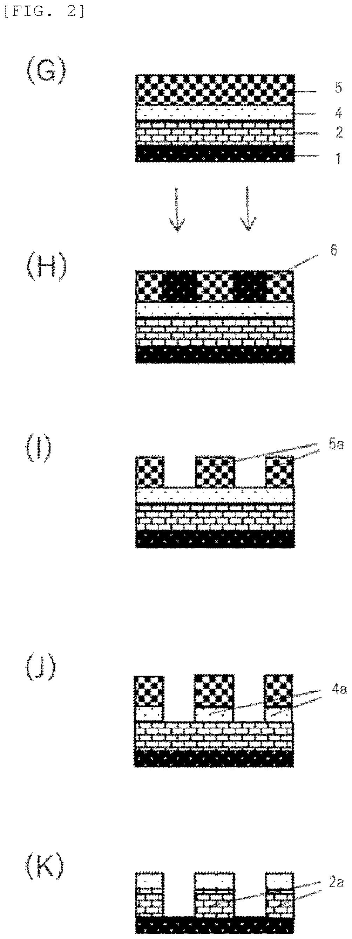

Furthermore, an example of the patterning process with the above two-layer resist process will be described with reference to FIG. 2. In this example, firstly, a metal-containing film 4 is formed on a layer to be processed 2 of a substrate to be processed 10 using the composition for forming a metal-containing film of the present invention, the substrate to be processed 10 consisting of a substrate 1 and the layer to be processed 2 stacked thereon; and a resist upper layer film 5 is formed on the metal-containing film 4 using a photoresist material, as illustrated in FIG. 2(G). Then, an exposed portion 6 of the resist upper layer film is subjected to pattern exposure as illustrated in FIG. 2(H). Next, by development using a developer, a resist upper layer film pattern 5a is formed in the resist upper layer film as illustrated in FIG. 2(I). Next, the pattern is transferred to the metal-containing film 4 by dry etching while using the resist upper layer film having the formed pattern 5a as a mask to obtain a metal-containing film pattern 4a as illustrated in FIG. 2(J). Then, the layer to be processed 2 on the substrate 1 is processed while using the metal-containing film having the formed pattern 4a as a mask to form a pattern 2a in the substrate to be processed 10 as illustrated in FIG. 2(K).

### EXAMPLE

Hereinafter, the present invention will be more specifically described with reference to Synthesis Examples, Comparative Synthesis Examples, Examples, and Comparative Examples. However, the present invention is not limited thereto.

### (A) Synthesis Examples of Compound for Forming Metal-Containing Film

In the following Synthesis Examples, an organic group-raw material group G: (G1) to (G15) and a silicon-containing organic group-raw material group H: (H1) to (H2) as described below were used.

The raw material group G: (G1) to (G15) and the raw material group H: (H1) to (H2) are shown below.

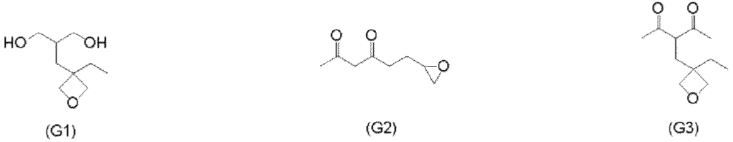

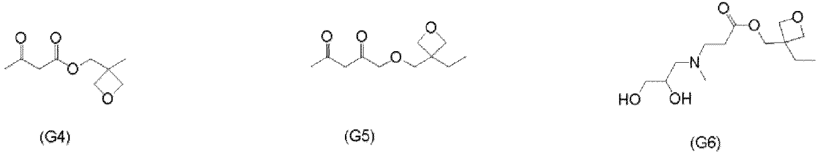

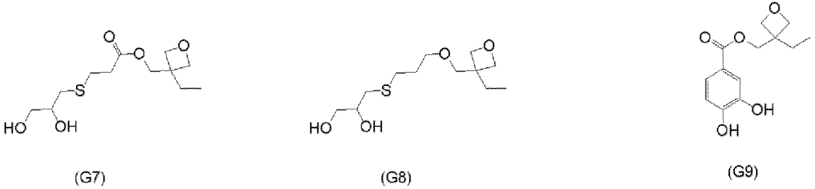

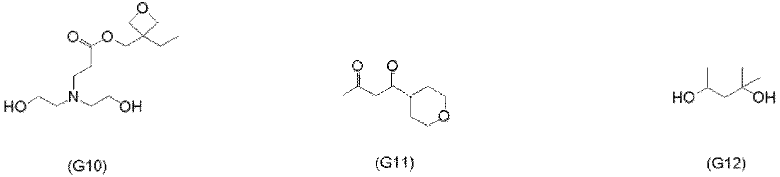

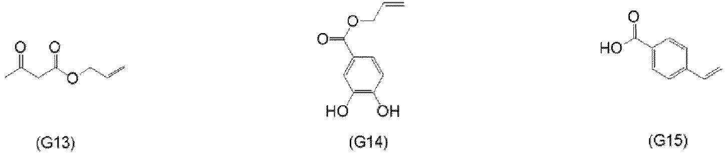

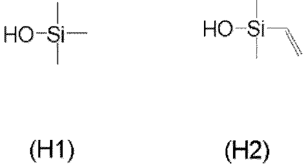

The following metal compounds were used in a metal source M.
(M1): titanium tetraisopropoxide (Sigma-Aldrich Corp., 377996)
(M2): Hf(OBu)₄: Hafnium(IV) n-butoxide (Sigma-Aldrich Corp., 667943)
(M3): Zr(OBu)₄: Zirconium(IV) tetrabutoxide (80% by mass of a 1-butanol solution) (Tokyo Chemical Industry Co., Ltd., Z0016)

### Synthesis Example 1: Synthesis of Compound for Forming Metal-Containing Film (A-1)

Under a nitrogen atmosphere, 28.4 g of titanium tetraisopropoxide (M1) was dissolved in 50.0 g of 2-propanol, and 50.0 g of an IPA solution containing 2.7 g of deionized water was added dropwise at a room temperature over 2 hours while being stirred. 15.8 g of the organic group-raw material group (G1) was added to the solution thus obtained and stirred at a room temperature for 30 minutes. After concentrating this solution under reduced pressure at 30°C, the solution was further heated to 60°C and continued to be heated under reduced pressure until no distillate was produced any more. When the distillate was no longer observed, 120.0 g of PGMEA/PGME (weight ratio: 70/30) solution was added and heated at 40°C under reduced pressure until IPA was no longer distilled out, thereby obtaining a PGMEA/PGME solution of the compound for forming a metal-containing film (A-1).

### Synthesis of Compounds (A-2) to (A-3)

The compounds (A-2) to (A-3) shown in Table 1 were obtained under the same reaction conditions as in Synthesis Example 1 except that the aforementioned metal source M and compound group G were used in the preparation amounts shown in Table 1.

**Table 1**

| Synthesis Example | Metal Raw Material | | Compound Group G | | Compound |
|---|---|---|---|---|---|
| | Raw Material | g | Raw Material | g | |
| 1 | M1 | 28.4 | G1 | 15.8 | A-1 |
| 2 | M2 | 48.5 | G2 | 15.6 | A-2 |
| 3 | M3 | 47.9 | G3 | 19.8 | A-3 |

### Synthesis Example 2: Synthesis of Compound for Forming Metal-Containing Film (A-4)

Under a nitrogen atmosphere, 47.9 g of zirconium(IV) tetrabutoxide (M3) was dissolved in 65.9 g of a PGMEA/PGME (weight ratio: 70/30) solution, and the reaction temperature was increased to 60°C while being stirred. A mixture in which 18.6 g of the compound G4 is suspended in 27.5 g of a PGMEA/PGME (weight ratio: 70/30) solution was added to the reaction system, and stirring was continued for 1 hour while the reaction temperature was kept at 60°C. After cooling to a room temperature, the reaction solution thus obtained was filtered through a 0.45 µm PTFE filter to obtain a PGMEA/PGME solution of the compound for forming a metal-containing film (A-4).

### Synthesis of Compounds (A-5) to (A-11)

The compounds (A-5) to (A-11) shown in Table 2 were obtained under the same reaction conditions as in Synthesis Example 2 except that the aforementioned metal source M and compound group G were used in the preparation amounts shown in Table 2.

**Table 2**

| Synthesis Example | Metal Raw Material | | Compound Group G | | Compound |
|---|---|---|---|---|---|
| | Raw Material | g | Raw Material | g | |
| 4 | M3 | 47.9 | G4 | 18.6 | A-4 |
| 5 | M3 | 47.9 | G5 | 21.4 | A-5 |
| 6 | M3 | 47.9 | G6 | 27.5 | A-6 |
| 7 | M3 | 47.9 | G7 | 27.8 | A-7 |
| 8 | M3 | 47.9 | G8 | 26.4 | A-8 |
| 9 | M3 | 47.9 | G9 | 25.2 | A-9 |
| 10 | M3 | 47.9 | G10 | 27.5 | A-10 |
| 11 | M3 | 47.9 | G11 | 17.0 | A-11 |

### Synthesis Example 3: Synthesis of Compound for Forming Metal-Containing Film (A-12)

Under a nitrogen atmosphere, 12.0 g of zirconium(IV) tetrabutoxide (M3) was dissolved in 16.5 g of a PGMEA/PGME (weight ratio: 70/30) solution, the reaction temperature was increased to 50°C while being stirred, and 6.8 g of the compound H1 was added dropwise to the solution. Thereafter, the reaction temperature was set to 60°C and stirring was continued for 2 hours. Next, a mixture in which 5.0 g of the compound G3 is suspended in 6.8 g of a PGMEA/PGME (weight ratio: 70/30) solution was added to the reaction system, and stirring was continued for 1 hour while the reaction temperature was kept at 60°C. After cooling to a room temperature, the reaction solution thus obtained was filtered through a 0.45 µm PTFE filter to obtain a PGMEA/PGME solution of the compound for forming a metal-containing film (A-12).

### Synthesis of Compounds (A-13) to (A-14) and Comparative Example Compounds (R-1) to (R-4)

The compounds (A-13) to (A-14) and comparative example compounds (R-1) to (R-4) shown in Table 3 were obtained under the same reaction conditions as in Synthesis Example 3, except that the aforementioned metal source M, compound group G, and compound group H were used in the preparation amounts shown in Table 3.

**Table 3**

| Synthesis Example | Metal Raw Material | | Compound Group G | | Compound Group H | | Compound |
|---|---|---|---|---|---|---|---|
| | Raw Material | g | Raw Material | g | Raw Material | g | |
| 12 | M3 | 12.0 | G3 | 5.0 | H1 | 6.8 | A-12 |
| 13 | M3 | 12.0 | G6 | 6.9 | H2 | 5.1 | A-13 |
| 14 | M3 | 12.0 | G7 | 7.0 | H1 | 4.5 | A-14 |
| Comparative Example 1 | M3 | 12.0 | G12 | 3.0 | H1 | 4.5 | R-1 |
| Comparative Example 2 | M3 | 12.0 | G13 | 3.6 | H1 | 4.5 | R-2 |
| Comparative Example 3 | M3 | 12.0 | G14 | 4.9 | H1 | 4.5 | R-3 |
| Comparative Example 4 | M3 | 12.0 | G15 | 7.4 | H1 | 4.5 | R-4 |

### Preparation of Composition for Forming Metal-Containing Film (MUL-1 to 19, Comparative MUL-1 to 4)

Used for preparation of the composition for forming a metal-containing film were the above compounds for forming a metal-containing film (A-1) to (A-14), (AG1) as the thermal acid generator, (AG2) as the photoacid generator, (XL1) to (XL2) as the crosslinking agent, and (F1: ethylene glycol dibenzyl ether, boiling point: 364°C) as the high boiling point solvent. After dissolving them in an organic solvent in the proportions shown in Table 4, the resultant was filtered through a 0.1 um filter made of a fluororesin, thereby preparing the compositions for forming a metal-containing film (MUL-1 to 19, Comparative MUL-1 to 4), respectively.

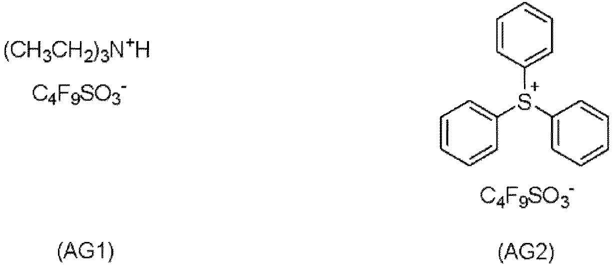

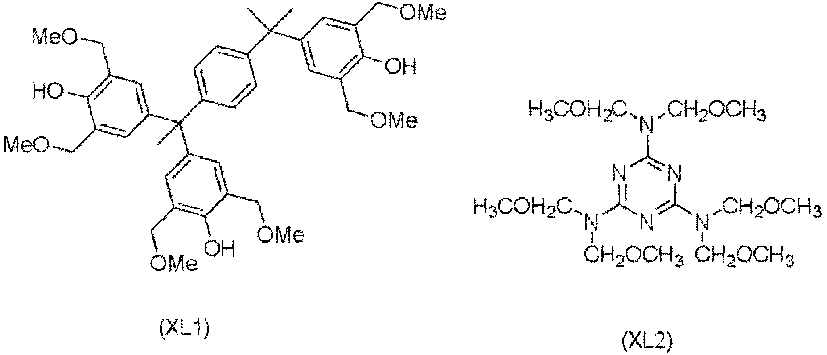

**Table 4**

| Composition for Forming Metal-Containing Film | (A) Compound for Forming Metal-Containing Film | Thermal Acid Generator | Photoacid Generator | Crosslinking Agent | High Boiling Point Solvent | Solvent |
|---|---|---|---|---|---|---|
| | (parts by mass) | (parts by mass) | (parts by mass) | (parts by mass) | (parts by mass) | (parts by mass) |
| MUL-1 | A-1(3) | - | - | - | - | PGMEA/PGME (67/30) |
| MUL-2 | A-2(3) | - | - | - | - | PGMEA/PGME (67/30) |
| MUL-3 | A-3(3) | - | - | - | - | PGMEA/PGME (67/30) |
| MUL-4 | A-4(3) | - | - | - | - | PGMEA/PGME (67/30) |
| MUL-5 | A-5(3) | - | - | - | - | PGMEA/PGME (67/30) |
| MUL-6 | A-6(3) | - | - | - | - | PGMEA/PGME (67/30) |
| MUL-7 | A-7(3) | - | - | - | - | PGMEA/PGME (67/30) |
| MUL-8 | A-8(3) | - | - | - | - | PGMEA/PGME (67/30) |
| MUL-9 | A-9(3) | - | - | - | - | PGMEA/PGME (67/30) |
| MUL-10 | A-10(3) | - | - | - | - | PGMEA/PGME (67/30) |
| MUL-11 | A-11(3) | - | - | - | - | PGMEA/PGME (67/30) |
| MUL-12 | A-12(3) | - | - | - | - | PGMEA/PGME (67/30) |
| MUL-13 | A-13(3) | - | - | - | - | PGMEA/PGME (67/30) |
| MUL-14 | A-14(4) | - | - | - | - | PGMEA/PGME (67/30) |
| MUL-15 | A-7(3) | AG1(0.2) | - | - | - | PGMEA/PGME (67/30) |
| MUL-16 | A-7(3) | - | AG2(0.2) | - | - | PGMEA/PGME (67/30) |
| MUL-17 | A-7(3) | AG1(0.2) | - | XL1(0.5) | - | PGMEA/PGME (67/30) |
| MUL-18 | A-7(3) | AG1(0.2) | - | XL2(0.5) | - | PGMEA/PGME (67/30) |
| MUL-19 | A-7(3) | - | - | - | F1(5) | PGMEA/PGME (67/30) |
| Comp. Ex. MUL-1 | R-1(3) | - | - | - | - | PGMEA/PGME (67/30) |
| Comp. Ex. MUL-2 | R-2(3) | - | - | - | - | PGMEA/PGME (67/30) |
| Comp. Ex. MUL-3 | R-3(3) | - | - | - | - | PGMEA/PGME (67/30) |
| Comp. Ex. MUL-4 | R-4(3) | - | - | - | - | PGMEA/PGME (67/30) |

### Example 1: Contact Angle Evaluation

The composition for forming a metal-containing film (MUL-1 to 19, Comparative Examples MUL-1 to 4) as prepared above was applied onto a silicon substrate and baked at 250°C for 60 seconds to form a metal-containing film having film thickness of 45 nm. For the respective cured films, a contact angle (CA1) with pure water was measured.

The results are shown in Table 5 below.

**Table 5**

| Example | Composition for Forming Metal-Containing Film | Contact Angle (CA1) |
|---|---|---|
| | | deg. |
| Example 1-1 | MUL-1 | 62 |
| Example 1-2 | MUL-2 | 63 |
| Example 1-3 | MUL-3 | 63 |
| Example 1-4 | MUL-4 | 64 |
| Example 1-5 | MUL-5 | 66 |
| Example 1-6 | MUL-6 | 63 |
| Example 1-7 | MUL-7 | 69 |
| Example 1-8 | MUL-8 | 68 |
| Example 1-9 | MUL-9 | 68 |
| Example 1-10 | MUL-10 | 62 |
| Example 1-11 | MUL-11 | 60 |
| Example 1-12 | MUL-12 | 63 |
| Example 1-13 | MUL-13 | 63 |
| Example 1-14 | MUL-14 | 69 |
| Example 1-15 | MUL-15 | 71 |
| Example 1-16 | MUL-16 | 70 |
| Example 1-17 | MUL-17 | 72 |
| Example 1-18 | MUL-18 | 73 |
| Example 1-19 | MUL-19 | 69 |
| Comparative Example 1-1 | Comparative Example MUL-1 | 35 |
| Comparative Example 1-2 | Comparative Example MUL-2 | 39 |
| Comparative Example 1-3 | Comparative Example MUL-3 | 50 |
| Comparative Example 1-4 | Comparative Example MUL-4 | 44 |

As shown in Table 5, it was found that all the compositions for forming a metal-containing film of the present invention (MUL-1 to 19) having the cyclic ether structure resulted in a film exhibiting the contact angle of greater than 60° with pure water, and thus good adhesiveness to the resist pattern can be expected. In particular, it was found that the composition for forming a metal-containing film using the compound having an epoxy or oxetane structure exhibited the high contact angle. Furthermore, it was found that Examples 1-15 and 1-17 to 1-18 using the composition containing the thermal acid generator (AG1) exhibited the high contact angle. Though the effect thereof is not certain, it is presumed that curing due to a ring opening reaction of the epoxy or oxetane structure was facilitated by an action of the acid generator.

On the other hand, the compositions for forming a metal-containing film in Comparative Examples (Comparative Examples MUL-1 to 4) with no cyclic ether structure showed results of the low contact angle, and thus insufficient adhesiveness to the resist pattern was expected.

### Example 2: Optical Constant (n/k) Evaluation

The composition for forming a metal-containing film (MUL-2 to 19, Comparative Examples MUL-1 to 4) was applied onto a silicon substrate and baked at 250°C for 60 seconds. Thereafter, an optical constant (n: refractive index, k: extinction coefficient) at a wavelength of 193 nm was determined using a variable incidence angle spectroscopic ellipsometer (VASE) manufactured by J. A. Woollam Company. The results are together shown in Table 6.

**Table 6**

| Example | Composition for Forming Metal-Containing Film | n | k |
|---|---|---|---|
| | | 193 nm | 193 nm |
| Example 2-1 | MUL-2 | 1.92 | 0.10 |
| Example 2-2 | MUL-3 | 1.90 | 0.19 |
| Example 2-3 | MUL-4 | 1.90 | 0.18 |
| Example 2-4 | MUL-5 | 1.89 | 0.19 |
| Example 2-5 | MUL-6 | 1.89 | 0.19 |
| Example 2-6 | MUL-7 | 1.92 | 0.18 |
| Example 2-7 | MUL-8 | 1.91 | 0.19 |
| Example 2-8 | MUL-9 | 1.83 | 0.34 |
| Example 2-9 | MUL-10 | 1.88 | 0.18 |
| Example 2-10 | MUL-11 | 1.87 | 0.20 |
| Example 2-11 | MUL-12 | 1.90 | 0.18 |
| Example 2-12 | MUL-13 | 1.89 | 0.19 |
| Example 2-13 | MUL-14 | 1.92 | 0.18 |
| Example 2-14 | MUL-15 | 1.92 | 0.18 |
| Example 2-15 | MUL-16 | 1.92 | 0.18 |
| Example 2-16 | MUL-17 | 1.91 | 0.19 |
| Example 2-17 | MUL-18 | 1.91 | 0.18 |
| Example 2-18 | MUL-19 | 1.92 | 0.18 |
| Comparative Example 2-1 | Comparative Example MUL-1 | 2.10 | 0.30 |
| Comparative Example 2-2 | Comparative Example MUL-2 | 2.02 | 0.28 |
| Comparative Example 2-3 | Comparative Example MUL-3 | 1.61 | 0.40 |
| Comparative Example 2-4 | Comparative Example MUL-4 | 1.73 | 0.50 |

As shown in Table 6, all the compositions for forming a metal-containing film of the present invention (MUL-2 to 19) containing hafnium and zirconium had the n value of 1.8 or greater and the k value of 0.1 to 0.5. Although depending on film thickness, a type of the upper layer film, etc., the optical constant generally in the range of the n value of 1.8 to 2.3 and the k value of 0.1 to 0.5 can significantly suppress reflected light from a substrate in ArF immersion/High-NA exposure even if a metal-containing film is formed to have thin film thickness of 20 nm or less, thereby allowing application of the metal-containing film as an underlayer film for photoresist patterning. In the above Examples, it was found that all the optical constants were within the suitable range, thereby allowing application thereof to photoresist patterning as a resist underlayer film. On the other hand, it is expected that in Comparative Examples 2-3 and 2-4, maybe because the metal-containing film contained a lot of organic components, the n value was 1.8 or less, resulting in insufficient performance for suppressing reflected light from a substrate in ArF immersion/High-NA exposure.

### Example 3: Etching Resistance Evaluation

The composition for forming a metal-containing film (MUL-1 to 19 and Comparative Examples MUL-1 to 4) was applied onto a silicon substrate and heated at 250°C for 60 seconds by using a hot plate to form a metal-containing film having film thickness of 50 nm (film thickness a). In Comparative Example 3-5, the below silicon atom-containing resist middle layer film material (SOG-1) was applied and baked at 220°C for 60 seconds to form a resist middle layer film having film thickness of 50 nm.

Regarding the silicon atom-containing resist middle layer film material (SOG-1), a polymer designated as an ArF silicon-containing middle layer film polymer (SiP1) and a thermal crosslinking catalyst (CAT1) were dissolved in a solvent containing 0.1% by mass of FC-4430 (manufactured by Sumitomo 3M Limited) in the proportions shown in Table 7, and filtered through a filter made of a fluororesin with a pore diameter of 0.1 um, thereby preparing the silicon atom-containing resist middle layer film material (SOG-1).

**Table 7**

| | Polymer | Thermal Crosslinking Catalyst | Solvent 1 | Solvent 2 |
|---|---|---|---|---|
| | (parts by mass) | (parts by mass) | (parts by mass) | (parts by mass) |
| SOG-1 | SiP1 | CAT1 | Propylene Glycol Monoethyl Ether | Pure Water |
| | (100) | (1) | (2900) | (400) |

Structural formulae of the ArF silicon-containing middle layer film polymer (SiP1) and the thermal crosslinking catalyst (CAT1) for use are shown below.

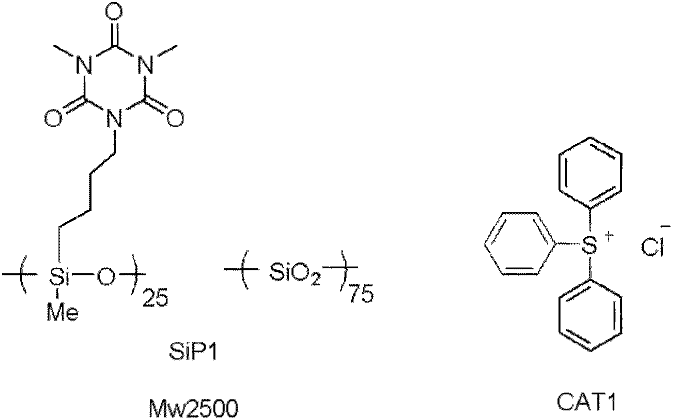

In Comparative Example 3-6, the below organic resist underlayer film material (SOC-1) was applied and baked at 350°C for 60 seconds to form a resist underlayer film having film thickness of 50 nm.

Regarding the organic resist underlayer film material (SOC-1), a polymer designated as an organic underlayer film polymer (CP1) was dissolved in a solvent containing 0.1% by mass of FC-4430 (manufactured by Sumitomo 3M Limited) in the proportions shown in Table 8, and filtered through a filter made of a fluororesin with a pore diameter of 0.1 um, thereby preparing the organic resist underlayer film material (SOC-1).

**Table 8**

| | Polymer | Solvent 1 |
|---|---|---|
| | (parts by mass) | (parts by mass) |
| SOC-1 | CP1 | Propylene Glycol Monoethyl Ether |
| | (100) | (3000) |

The structural formula of the organic underlayer film polymer (CP1) for use is shown below. 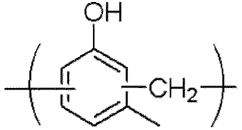 Mw=7,000, Mw/Mn=3.50

Next, etching was performed using CF₄ gas and O₂ gas, respectively, under the following conditions using an etching system CE-300I manufactured by ULVAC, Inc. and film thickness b was measured. Based on film thickness etched for a specified time period using each gas (film thickness b - film thickness a), film thickness to be etched for one minute was calculated as an etch rate (nm/min).

The etch rate for the CF₄ gas of 20 nm/min or less was determined as "A (excellent)", 20 nm/min to 30 nm/min as "B (good)", and 30 nm/min or greater as "C (poor)". The results are shown in Table 9.

The etch rate for the O₂ gas of 20 nm/min or less was determined as "A (excellent)", 20 nm/min to 30 nm/min as "B (good)", and 30 nm/min or greater as "C (poor)". The results are shown in Table 9.

### Dry etching conditions with CF₄ gas

Pressure: 3 Pa
Antenna RF power: 100 W
Bias RF power: 15 W
CF₄ gas flow rate: 15 sccm
Time: 30 sec

### Dry etching conditions with O₂ gas

Pressure: 3 Pa
Antenna RF power: 300 W
Bias RF power: 10 W
O₂ gas flow rate: 25 sccm
Time: 30 sec

**Table 9**

| Example | Composition for Forming Metal-Containing Film | Etch Rate (nm/min) | Etch Rate (nm/min) |
|---|---|---|---|
| | | CF4 | O2 |
| Example 3-1 | MUL-1 | A | A |
| Example 3-2 | MUL-2 | A | A |
| Example 3-3 | MUL-3 | A | A |
| Example 3-4 | MUL-4 | A | A |
| Example 3-5 | MUL-5 | A | A |
| Example 3-6 | MUL-6 | A | A |
| Example 3-7 | MUL-7 | A | A |
| Example 3-8 | MUL-8 | A | A |
| Example 3-9 | MUL-9 | A | A |
| Example 3-10 | MUL-10 | A | A |
| Example 3-11 | MUL-11 | A | A |
| Example 3-12 | MUL-12 | A | A |
| Example 3-13 | MUL-13 | A | A |
| Example 3-14 | MUL-14 | A | A |
| Example 3-15 | MUL-15 | A | A |
| Example 3-16 | MUL-16 | A | A |
| Example 3-17 | MUL-17 | A | A |
| Example 3-18 | MUL-18 | A | A |
| Example 3-19 | MUL-19 | A | A |
| Comparative Example 3-1 | Comparative Example MUL-1 | A | A |
| Comparative Example 3-2 | Comparative Example MUL-2 | A | A |
| Comparative Example 3-3 | Comparative Example MUL-3 | B | B |
| Comparative Example 3-4 | Comparative Example MUL-4 | B | B |
| Comparative Example 3-5 | SOG-1 | C | B |
| Comparative Example 3-6 | SOC-1 | B | C |

As shown in Table 9, it was found that all the compositions for forming a metal-containing film of the present invention (MUL-1 to 19) showed excellent etching resistance to the CF₄ gas and O₂ gas. It can be deemed that the compositions for forming a metal-containing film of the present invention are excellent in resistance against etching using the O₂ gas compared to SOG-1, which was used in Comparative Example 3-5, and therefore are suitable for the three-layer resist process where the resist pattern is transferred to the substrate to be processed, in combination with the organic resist underlayer film. It can be deemed that the compositions for forming a metal-containing film of the present invention are excellent in resistance against etching using the CF₄ gas compared to SOC-1, which was used in Comparative Example 3-6, and therefore are suitable for the two-layer resist process where the resist pattern is transferred to the substrate to be processed, in combination with the photoresist upper layer film.

### Example 4: Patterning Process

The above SOC-1 was applied onto a Si wafer by spin coating and baked at 350°C for 60 seconds to form a carbon film having film thickness of 60 nm as an organic underlayer film on a substrate. The carbon proportion in the carbon film was 46 atomic% and the optical constant (n/k) at a wavelength of 193 nm was 1.42/0.50. A composition obtained by diluting the composition for forming a metal-containing film (MDL-3 to 19 and Comparative Examples MDL-1 to 4) with an organic solvent (PGMEA/PGME=700/300 parts by mass) was applied thereon and heated at 250°C for 60 seconds using a hot plate to form a resist middle layer film having film thickness of 15 nm. A single-layer resist for ArF as the resist upper layer film material was applied thereon and baked at 105°C for 60 seconds to form a photoresist film having film thickness of 100 nm. A liquid immersion topcoat material (TC-1) was applied onto the photoresist film and baked at 90°C for 60 seconds to form a topcoat having film thickness of 50 nm.

A polymer (RP1), an acid generator (PAG1), and a basic compound (Amine1) were dissolved in a solvent containing 0.1% by mass of a surfactant FC-4430 (manufactured by Sumitomo 3M Limited) in the proportions shown in Table 10, and filtered through a 0.1 um filter made of a fluororesin, thereby preparing the resist upper layer film material (single-layer resist for ArF).

**Table 10**

| | Polymer (parts by mass) | Acid Generator (parts by mass) | Basic Compound (parts by mass) | Solvent (parts by mass) |
|---|---|---|---|---|
| Single-Layer Resist for ArF | RP1 (100) | PAG1 (6.6) | Amine1 (0.8) | PGMEA (2,500) |

The polymer (RP1), the acid generator (PAG1), and the basic compound (Amine1) used in the resist upper layer film material (single-layer resist for ArF) are shown below.

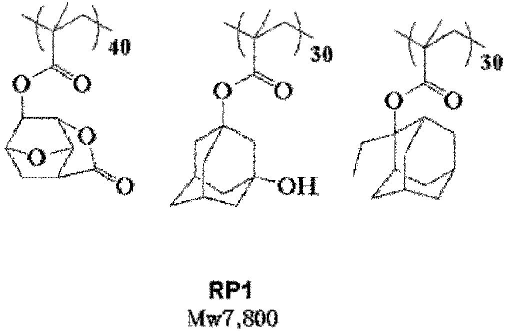

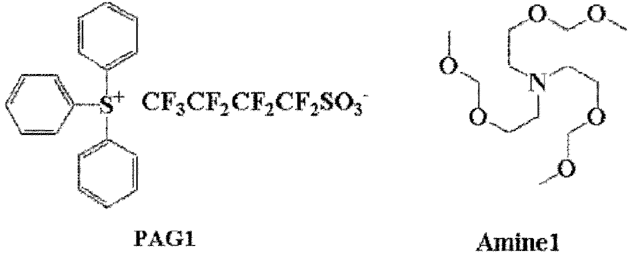

The liquid immersion topcoat material (TC-1) was prepared by dissolving a topcoat polymer (PP1) in an organic solvent in the proportion shown in Table 11 and filtering through a 0.1 um filter made of a fluororesin.

**Table 11**

| | Topcoat Polymer (parts by mass) | Organic Solvent (parts by mass) |
|---|---|---|
| TC-1 | PP1 (100) | diisoamyl ether (2,700) |
| | | 2-methyl-1-butanol (270) |

The topcoat polymer (PP1) used in the liquid immersion topcoat material (TC-1) is shown below.

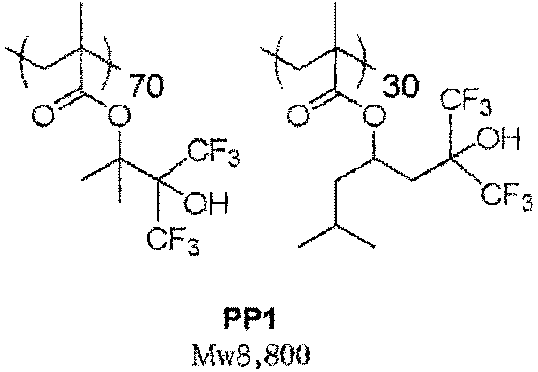

Next, the photoresist film was exposed using an ArF immersion lithography system (manufactured by Nikon Corporation; NSR-S610C, NA1.30, σ0.98/0.78, 35° s-polarized dipole illumination, 6% half-tone phase-shifting mask), baked (PEB) at 100°C for 60 seconds, and developed for 30 seconds using a 2.38% by mass tetramethylammonium hydroxide (TMAH) aqueous solution, thereby obtaining a 50 nm 1:1 positive line-and-space pattern (resist upper layer film pattern).

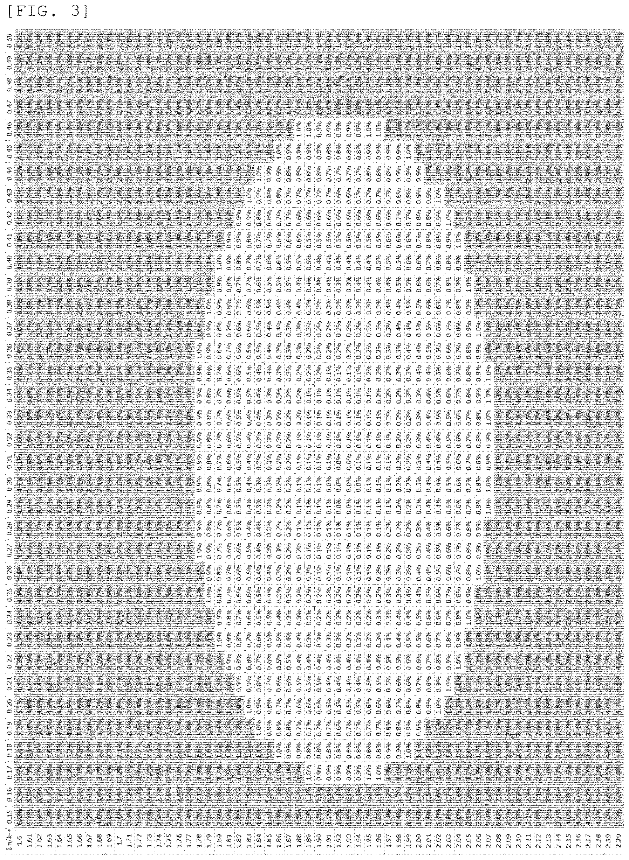

The reflectance was calculated using PROLITH 2020a (Litho Tech Japan Corporation). The film thickness of the metal-containing film was fixed at 10 nm, and the reflectance was calculated when the n and k of the metal-containing film were 1.60 to 2.20 and 0.15 to 0.50, respectively. The results thereof are shown in FIG. 3. The optical constant (n/k) is preferable that enables reflected light from the underlying substrate to be reduced to 1.0% or lower during pattern exposure. The pattern cross section was observed using S-4700 (electron microscope manufactured by Hitachi Ltd.).

The results are shown in Table 12.

Next, the resist middle layer film was etched by dry etching while using the resist upper layer film pattern as a mask to form a hard mask pattern; the organic underlayer film was etched while using the hard mask pattern thus obtained as a mask to form a pattern; and SiO₂ film was etched while using the resist underlayer film pattern thus obtained as a mask. The etching conditions were as follows.

The pattern cross section was observed using S-4700 (electron microscope manufactured by Hitachi Ltd.). The results are shown in Table 12.

Conditions for transferring the hard mask pattern to the metal-containing film (resist middle layer film).

### Dry etching conditions with Cl₂ gas

Pressure: 1 Pa
Antenna RF power: 320 W
Bias RF power: 30 W
Cl₂ gas flow rate: 25 sccm
Time: 15 sec

Conditions for transferring the resist middle layer film pattern to the organic underlayer film.

### Dry etching conditions with O₂ gas

Pressure: 1 Pa
Antenna RF power: 300 W
Bias RF power: 10 W
O₂ gas flow rate: 25 sccm
Time: 20 sec

Conditions for transferring the resist underlayer film pattern to the SiO₂ film.

### Dry etching conditions with CF₄ gas

Pressure: 1 Pa
Antenna RF power: 100 W
Bias RF power: 15 W
CF₄ gas flow rate: 15 sccm
Time: 60 sec

**Table 12**

| Example | Composition for Forming Metal-Containing Film | Reflectance | After Development | After Transfer to Substrate by Etching |
|---|---|---|---|---|
| | | % | Pattern Shape | Pattern Shape |
| Example 4-1 | MUL-3 | 0.7% | Vertical Profile | Vertical profile |
| Example 4-2 | MUL-4 | 0.8% | Vertical Profile | Vertical Profile |
| Example 4-3 | MUL-5 | 0.7% | Vertical Profile | Vertical Profile |
| Example 4-4 | MUL-6 | 0.7% | Vertical Profile | Vertical Profile |
| Example 4-5 | MUL-7 | 0.8% | Vertical Profile | Vertical Profile |
| Example 4-6 | MUL-8 | 0.7% | Vertical Profile | Vertical Profile |
| Example 4-7 | MUL-9 | 0.5% | Vertical Profile | Vertical Profile |
| Example 4-8 | MUL-10 | 0.9% | Vertical Profile | Vertical Profile |
| Example 4-9 | MUL-11 | 0.7% | Vertical Profile | Vertical Profile |
| Example 4-10 | MUL-12 | 0.8% | Vertical Profile | Vertical Profile |
| Example 4-11 | MUL-13 | 0.7% | Vertical Profile | Vertical Profile |
| Example 4-12 | MUL-14 | 0.8% | Vertical Profile | Vertical Profile |
| Example 4-13 | MUL-15 | 0.8% | Vertical Profile | Vertical Profile |
| Example 4-14 | MUL-16 | 0.8% | Vertical Profile | Vertical Profile |
| Example 4-15 | MUL-17 | 0.7% | Vertical Profile | Vertical Profile |
| Example 4-16 | MUL-18 | 0.8% | Vertical Profile | Vertical Profile |
| Example 4-17 | MUL-19 | 0.8% | Vertical Profile | Vertical Profile |
| Comparative Example 4-1 | Comparative Example MUL-1 | 1.4% | Pattern Collapse Observed | - |
| Comparative Example 4-2 | Comparative Example MUL-2 | 0.5% | Pattern Collapse Observed | - |
| Comparative Example 4-3 | Comparative Example MUL-3 | 3. 8% | Pattern Collapse Observed | - |
| Comparative Example 4-4 | Comparative Example MUL-4 | 2.5% | Pattern Collapse Observed | - |

As shown in Table 12, it was confirmed that all the compositions for forming a metal-containing film of the present invention (MUL-3 to 19) had good pattern shape after development. It is presumed that the good resist pattern was obtained because the metal-containing film material of the present invention contained the cyclic ether structure so as to achieve high adhesiveness to the resist pattern and had the optical constant enabling sufficient reduction in the reflected light from the substrate during pattern exposure. Furthermore, it was confirmed that after transfer to the substrate by etching, the resist upper layer film pattern was eventually transferred to the substrate favorably, and thus the compositions for forming a metal-containing film of the present invention (MUL-3 to 19) are suitable for use in the fine processing in the multilayer resist method. On the other hand, in the compositions for forming a metal-containing film (Comparative Examples MUL-1 to 4) that showed the results of the low contact angle in the contact angle evaluation in Example 1, collapse of the resist upper layer film pattern was observed after development.

Accordingly, the compound for forming a metal-containing film, the composition using the compound, and the patterning process of the present invention can achieve high adhesiveness to the resist upper layer film pattern and an effect of preventing collapse of a fine pattern, and also provide a pattern shape of the resist upper layer film having high rectangularity; therefore, they are particularly suitable for use in the multilayer resist process and extremely useful for fine patterning to manufacture a semiconductor device.

The present description encompasses the following inventions.
[1]: A compound for forming a metal-containing film, comprising: at least one metal atom selected from a group consisting of Ti, Zr, and Hf; and a multidentate ligand coordinated to the metal atom and containing a cyclic ether structure having 2 to 13 carbon atoms.
[2]: The compound for forming a metal-containing film according to [1], wherein the multidentate ligand has a structure represented by the following general formulae (1-A) to (1-D), 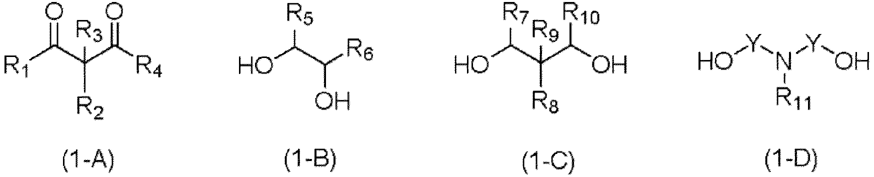 in the above general formulae (1-A) to (1-D), R₁ to R₄ represent a hydrogen atom or a monovalent organic group having 1 to 20 carbon atoms, which optionally contains a cyclic ether structure having 2 to 13 carbon atoms; R₅ to R₆ represent a hydrogen atom or a monovalent organic group having 1 to 20 carbon atoms, which optionally contains a cyclic ether structure having 2 to 13 carbon atoms; R₇ to R₁₀ represent a hydrogen atom or a monovalent organic group having 1 to 20 carbon atoms, which optionally contains a cyclic ether structure having 2 to 13 carbon atoms; R₁₁ represents a monovalent organic group having 1 to 20 carbon atoms, which optionally contains a cyclic ether structure having 2 to 13 carbon atoms; and Y represents a divalent organic group having 1 to 10 carbon atoms,
   wherein compounds represented by the above general formulae (1-A) to (1-D) contain at least one or more cyclic ether structures having 2 to 13 carbon atoms,
   in the above general formula (1-A), R₂ and R₃ optionally bond together to form a cyclic ether structure having 2 to 13 carbon atoms,
   in the above general formula (1-B), adjacent R₅ and R₆ optionally bond together to form an unsaturated or saturated ring structure, and
   in the above general formula (1-C), adjacent R₈ and R₉ optionally bond together to form a cyclic ether structure having 2 to 13 carbon atoms or an unsaturated or saturated ring structure.
[3]: The compound for forming a metal-containing film according to [2], wherein the multidentate ligand has the structure represented by the above formula (1-A) or (1-B).
[4]: The compound for forming a metal-containing film according to any one of [1] to [3], wherein the cyclic ether structure is represented by any of the following formulae (a-1) and (a-2), 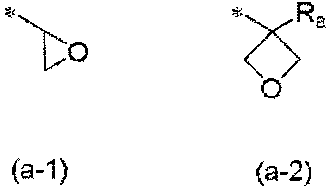 wherein Rₐ represents a hydrogen atom or an organic group having 1 to 10 carbon atoms; and * represents a bonding site.
[5]: The compound for forming a metal-containing film according to any one of [2] to [4], wherein the compound for forming a metal-containing film is a reaction product of a metal-containing compound containing any of a metal compound represented by the following formula (2) and a hydrolyzate, a condensate, or a hydrolysis condensate of the metal compound represented by the following formula (2), and the multidentate ligand having the structure represented by the above general formulae (1-A) to (1-D),

   LₐMX_{b} (2)

   wherein M represents any of Ti, Zr, and Hf; L represents a monodentate and/or multidentate ligand having 1 to 30 carbon atoms; X represents a hydrolyzable group selected from a halogen atom, an alkoxy group, a carboxylate group, an acyloxy group, and -NR^{a}R^{b}, wherein each of R^{a} and R^{b} independently represents a hydrogen atom or a monovalent organic group having 1 to 20 carbon atoms, satisfying a+b=4 wherein "a" and "b" represent an integer of 0 to 4.
[6]: The compound for forming a metal-containing film according to [5], wherein the metal compound represented by the formula (2) has a structure represented by the following formula (3),

   M(OR^{1A})₄ (3)

   wherein M represents Ti, Zr, or Hf; and R^{1A} represents a monovalent organic group having 1 to 20 carbon atoms.
[7]: The compound for forming a metal-containing film according to any one of [1] to [6], further comprising a ligand derived from a silicon compound represented by the following general formula (4), 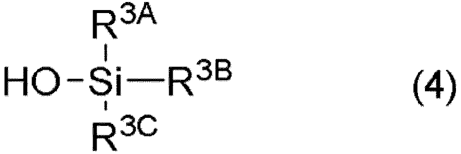 in the above general formula (4), R^{3A}, R^{3B}, and R^{3C} represent any organic group selected from an organic group having 1 to 30 carbon atoms, having a crosslinking group with a structure represented by any of the following general formulae (b-1) to (b-3), a substituted or unsubstituted alkyl group having 1 to 20 carbon atoms and aryl group having 1 to 20 carbon atoms; 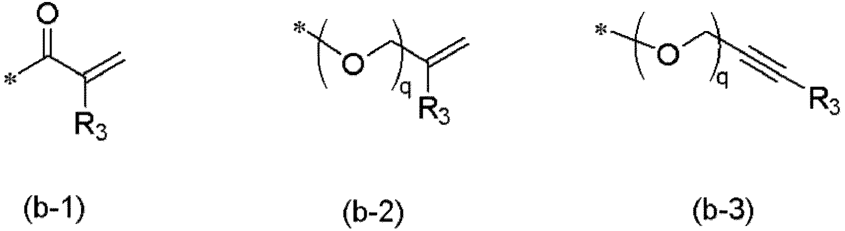 in the above general formulae (b-1) to (b-3), R₃ represents a hydrogen atom or a monovalent organic group having 1 to 10 carbon atoms; "q" represents 0 or 1; and * represents a bonding site.
[8]: A composition for forming a metal-containing film, comprising:
   (A) the compound for forming a metal-containing film according to any one of [1] to [7]; and
   (B) an organic solvent.
[9]: The composition for forming a metal-containing film according to [8], comprising one or more of (C) a thermal acid generator and (D) a photoacid generator.
[10]: The composition for forming a metal-containing film according to [8] or [9], further comprising one or more of (E) a crosslinking agent and (F) a surfactant.
[11]: The composition for forming a metal-containing film according to any one of [8] to [10], wherein (B) the organic solvent is a mixture of one or more kinds of organic solvents having a boiling point of less than 180°C and one or more kinds of organic solvents having a boiling point of 180°C or higher.
[12]: A patterning process for forming a pattern in a substrate to be processed, comprising the steps of:
   (I-1) applying the composition for forming a metal-containing film according to any one of [8] to [11] on a substrate to be processed and thereafter performing heat treatment to form a metal-containing film;
   (I-2) forming a resist upper layer film on the metal-containing film using a photoresist material;
   (I-3) forming a pattern in the resist upper layer film by developing the resist upper layer film using a developer after pattern exposure;
   (I-4) transferring the pattern to the metal-containing film by dry etching while using the resist upper layer film having the formed pattern as a mask; and
   (I-5) processing the substrate to be processed while using the metal-containing film having the formed pattern as a mask to form the pattern in the substrate to be processed.
[13]: A patterning process for forming a pattern in a substrate to be processed, comprising the steps of:
   (II-1) forming a resist underlayer film on a substrate to be processed;
   (II-2) applying the composition for forming a metal-containing film according to any one of [8] to [11] on the resist underlayer film and thereafter performing heat treatment to form a metal-containing film;
   (II-3) forming a resist upper layer film on the metal-containing film using a photoresist material;
   (II-4) forming a pattern in the resist upper layer film by developing the resist upper layer film using a developer after pattern exposure;
   (II-5) transferring the pattern to the metal-containing film by dry etching while using the resist upper layer film having the formed pattern as a mask;
   (II-6) transferring the pattern to the resist underlayer film by dry etching while using the metal-containing film having the formed pattern as a mask; and
   (II-7) processing the substrate to be processed while using the resist underlayer film having the formed pattern as a mask to form the pattern in the substrate to be processed.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A compound for forming a metal-containing film, comprising: at least one metal atom selected from a group consisting of Ti, Zr, and Hf; and a multidentate ligand coordinated to the metal atom and containing a cyclic ether structure having 2 to 13 carbon atoms.

2. The compound for forming a metal-containing film according to claim 1, wherein the multidentate ligand has a structure represented by the following general formulae (1-A) to (1-D), 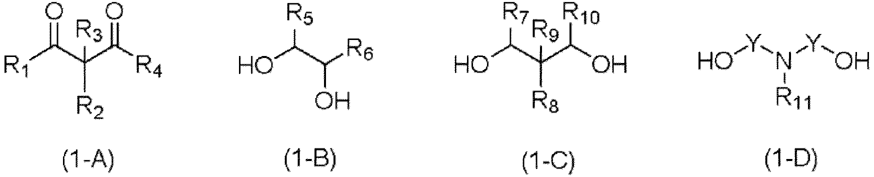
in the above general formulae (1-A) to (1-D), R₁ to R₄ represent a hydrogen atom or a monovalent organic group having 1 to 20 carbon atoms, which optionally contains a cyclic ether structure having 2 to 13 carbon atoms; R₅ to R₆ represent a hydrogen atom or a monovalent organic group having 1 to 20 carbon atoms, which optionally contains a cyclic ether structure having 2 to 13 carbon atoms; R₇ to R₁₀ represent a hydrogen atom or a monovalent organic group having 1 to 20 carbon atoms, which optionally contains a cyclic ether structure having 2 to 13 carbon atoms; R₁₁ represents a monovalent organic group having 1 to 20 carbon atoms, which optionally contains a cyclic ether structure having 2 to 13 carbon atoms; and Y represents a divalent organic group having 1 to 10 carbon atoms,
wherein compounds represented by the above general formulae (1-A) to (1-D) contain at least one or more cyclic ether structures having 2 to 13 carbon atoms,
in the above general formula (1-A), R₂ and R₃ optionally bond together to form a cyclic ether structure having 2 to 13 carbon atoms,
in the above general formula (1-B), adjacent R₅ and R₆ optionally bond together to form an unsaturated or saturated ring structure, and
in the above general formula (1-C), adjacent R₈ and R₉ optionally bond together to form a cyclic ether structure having 2 to 13 carbon atoms or an unsaturated or saturated ring structure.

3. The compound for forming a metal-containing film according to claim 2, wherein the multidentate ligand has the structure represented by the above formula (1-A) or (1-B).

4. The compound for forming a metal-containing film according to claim 1, wherein the cyclic ether structure is represented by any of the following formulae (a-1) and (a-2), 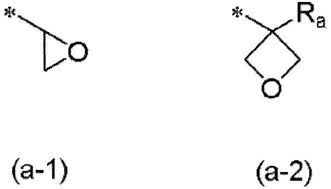 wherein Rₐ represents a hydrogen atom or an organic group having 1 to 10 carbon atoms; and * represents a bonding site.

5. The compound for forming a metal-containing film according to claim 2, wherein the compound for forming a metal-containing film is a reaction product of a metal-containing compound containing any of a metal compound represented by the following formula (2) and a hydrolyzate, a condensate, or a hydrolysis condensate of the metal compound represented by the following formula (2), and the multidentate ligand having the structure represented by the above general formulae (1-A) to (1-D),
LₐMX_{b} (2)
wherein M represents any of Ti, Zr, and Hf; L represents a monodentate and/or multidentate ligand having 1 to 30 carbon atoms; X represents a hydrolyzable group selected from a halogen atom, an alkoxy group, a carboxylate group, an acyloxy group, and -NR^{a}R^{b}, wherein each of R^{a} and R^{b} independently represents a hydrogen atom or a monovalent organic group having 1 to 20 carbon atoms, satisfying a+b=4 wherein "a" and "b" represent an integer of 0 to 4.

6. The compound for forming a metal-containing film according to claim 5, wherein the metal compound represented by the formula (2) has a structure represented by the following formula (3),
M(OR^{1A})₄ (3)
wherein M represents Ti, Zr, or Hf; and R^{1A} represents a monovalent organic group having 1 to 20 carbon atoms.

7. The compound for forming a metal-containing film according to claim 1, further comprising a ligand derived from a silicon compound represented by the following general formula (4), 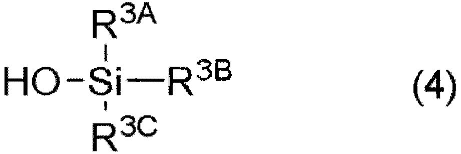 in the above general formula (4), R^{3A}, R^{3B}, and R^{3C} represent any organic group selected from an organic group having 1 to 30 carbon atoms, having a crosslinking group with a structure represented by any of the following general formulae (b-1) to (b-3), a substituted or unsubstituted alkyl group having 1 to 20 carbon atoms and aryl group having 1 to 20 carbon atoms; 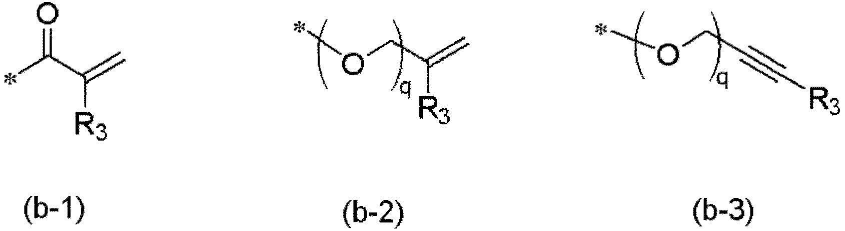 in the above general formulae (b-1) to (b-3), R₃ represents a hydrogen atom or a monovalent organic group having 1 to 10 carbon atoms; "q" represents 0 or 1; and * represents a bonding site.

8. A composition for forming a metal-containing film, comprising:
(A) the compound for forming a metal-containing film according to any one of claims 1 to 7; and
(B) an organic solvent.

9. The composition for forming a metal-containing film according to claim 8, comprising one or more of (C) a thermal acid generator and (D) a photoacid generator.

10. The composition for forming a metal-containing film according to claim 8, further comprising one or more of (E) a crosslinking agent and (F) a surfactant.

11. The composition for forming a metal-containing film according to claim 8, wherein (B) the organic solvent is a mixture of one or more kinds of organic solvents having a boiling point of less than 180°C and one or more kinds of organic solvents having a boiling point of 180°C or higher.

12. A patterning process for forming a pattern in a substrate to be processed, comprising the steps of:
(I-1) applying the composition for forming a metal-containing film according to claim 8 on a substrate to be processed and thereafter performing heat treatment to form a metal-containing film;
(I-2) forming a resist upper layer film on the metal-containing film using a photoresist material;
(I-3) forming a pattern in the resist upper layer film by developing the resist upper layer film using a developer after pattern exposure;
(I-4) transferring the pattern to the metal-containing film by dry etching while using the resist upper layer film having the formed pattern as a mask; and
(I-5) processing the substrate to be processed while using the metal-containing film having the formed pattern as a mask to form the pattern in the substrate to be processed.

13. A patterning process for forming a pattern in a substrate to be processed, comprising the steps of:
(II-1) forming a resist underlayer film on a substrate to be processed;
(II-2) applying the composition for forming a metal-containing film according to claim 8 on the resist underlayer film and thereafter performing heat treatment to form a metal-containing film;
(II-3) forming a resist upper layer film on the metal-containing film using a photoresist material;
(II-4) forming a pattern in the resist upper layer film by developing the resist upper layer film using a developer after pattern exposure;
(II-5) transferring the pattern to the metal-containing film by dry etching while using the resist upper layer film having the formed pattern as a mask;
(II-6) transferring the pattern to the resist underlayer film by dry etching while using the metal-containing film having the formed pattern as a mask; and
(II-7) processing the substrate to be processed while using the resist underlayer film having the formed pattern as a mask to form the pattern in the substrate to be processed.
